(19) Europäisches Patentamt / European Patent Office / Office européen des brevets

(11) **EP 4 344 639 B1**

(12) **EUROPEAN PATENT SPECIFICATION**

(45) Date of publication and mention
of the grant of the patent:
**27.08.2025  Bulletin 2025/35**

(21) Application number: **22198932.0**

(22) Date of filing: **30.09.2022**

(51) International Patent Classification (IPC):
*A61B 5/265* (2021.01)    *A61B 5/259* (2021.01)

(52) Cooperative Patent Classification (CPC):
**A61B 5/265; A61B 5/259;** A61B 2562/125

(54) **SILVER/SILVER CHLORIDE SENSOR ELEMENT**

SILBER/SILBERCHLORID-SENSORELEMENT

ELÉMENT DE DÉTECTION D'ARGENT/CHLORURE D'ARGENT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**03.04.2024  Bulletin 2024/14**

(73) Proprietor: **Ambu A/S
2750 Ballerup (DK)**

(72) Inventors:
• **HANSEN, Daniel
  2100 Copenhagen (DK)**
• **RIIS DAMMEYER, Marie
  2750 Ballerup (DK)**

(74) Representative: **Guardian
IP Consulting I/S
Diplomvej, Building 381
2800 Kgs. Lyngby (DK)**

(56) References cited:
US-A- 3 834 373        US-A1- 2005 261 565
US-A1- 2018 215 941    US-A1- 2019 000 337
US-B1- 10 898 094

## Description

The present disclosure relates to a medical electrode for biopotential monitoring and/or recording of biopotential signals when attached to the skin of a human or animal. The medical electrode comprises a sensor element and an ion conductive layer, which is in contact with the skin side of a sensor layer in the sensor element. The sensor layer comprises silver (Ag) and silver chloride (AgCl) and has a tailored effective AgCl area density, which depends on the electrode wear time and surface area of the sensor layer.

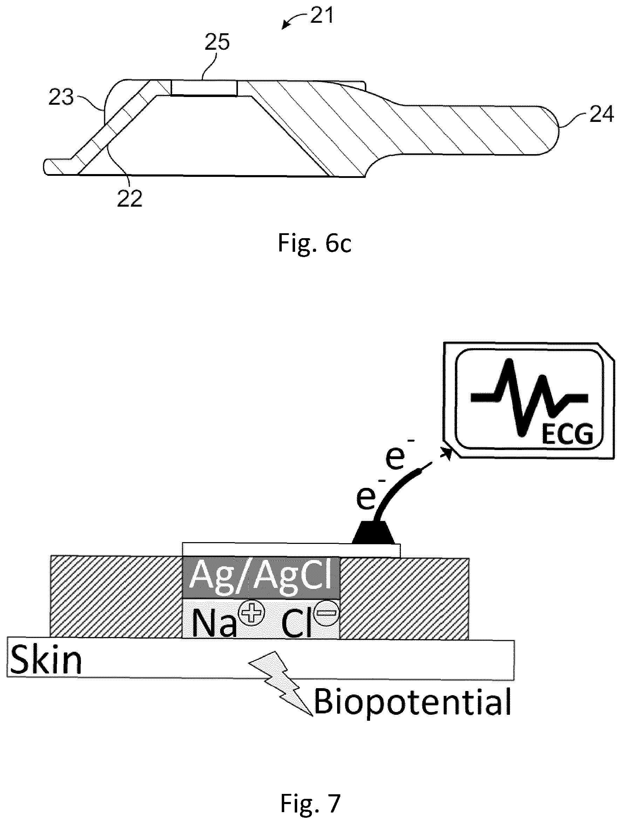

Recording and/or monitoring of biopotentials from the heart, skeletal muscles or the brain is important for diagnosis of diseases and inabilities such as heart arrhythmia, muscle disorders and epilepsy. For example, biopotentials from the heart can be detected through the skin using medical electrodes and can be displayed as an electrocardiogram (ECG). These ECG sensors are attached to the skin using skin adhesives and detect the ECG signal with an electrolyte gel, or ion conductive layer, and a sensor element. A typical ECG sensor is schematically shown in Figure 7.

Recording and/or monitoring of biopotentials from muscles is called electromyography (EMG) while recording and/or monitoring of biopotentials from the brain is called electroencephalography (EEG).

The ECG signal propagates through the physiological liquid as an ion polarization and can propagate to the ions in the electrolyte gel if sufficient contact between the gel and the skin is obtained. The polarization influences the electrochemical reaction on the surface of the sensor. The sensor surface layer typically comprises silver (Ag) and silver chloride (AgCl). The electrochemical reaction occurring at the sensor surface may be described as:

$$ Ag + Cl^- \rightleftharpoons AgCl + e^- \qquad\qquad (1) $$

Ag and AgCl are present on the sensor surface, whereas the chloride ions are included in the electrolyte gel. The electrons are a product of the electrochemical reaction and are transmitted to a monitor where the obtained signal can be processed, viewed and analyzed by healthcare professionals.

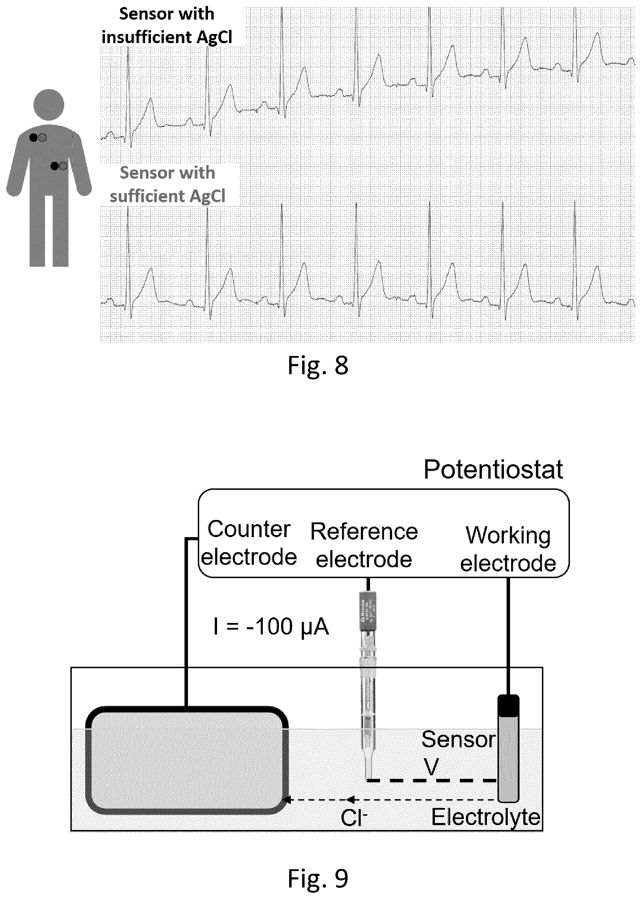

Medical electrodes in use are typically exposed to a leakage current from the hardware or the patient. According to the standard, ANSI AAMI EC12 (2015), the leakage current is assumed to be constant 200 nA. When the leakage current passes though the sensor element in the medical electrode, AgCl is consumed, whereby Ag is deposited as pure silver and chloride ions are released to the electrolyte gel. For short periods, this leakage current may seem insignificant, but for longer periods of several days or weeks, the leakage current may introduce an imbalance between the amount of AgCl and Ag in the sensor element, which may lead to baseline drifting and eventually electrode failure. In Figure 8 ECG signals obtained with insufficient AgCl (top) and sufficient AgCl (bottom) are shown. Unsatisfactory baseline drifting occurs in the top figure, i.e. when the ECG signals are recorded with a medical electrode having an insufficient amount of AgCl. Bioelectrical sensors designed to be single use electrodes are discarded as waste after use. If the bioelectrical sensors contain excessive amounts of silver in the sensor element, it may lead to loss of valuable and expensive silver being thrown in the waste without being recycled or reused. It is therefore important to consider the application time and the clinical application in order to tailor the medical electrode to the desired application and to utilize the silver optimally.

It is known from the prior art to make an effort to reduce the amount of expensive silver in Ag/AgCl sensor elements.

US2019000337A1 discloses a medical electrode with optimized amount of silver.

EP 2314345 B1 discloses a medical, self-adhesive, flexible disposable electrode for non-invasive supply or capture of electrical signals through the skin of a human or animal. The total silver content of the layer of Ag/AgCl in relation to the surface which has a conductive connection to the hydrogel is 0.02 to 0.15 mg/mm². The document further teaches that, in comparison thereto, the silver use in conventional commercial sleep laboratory electrodes is approximately 2.5 mg/mm². Accordingly, this document teaches that the important feature is the total content of Ag + AgCl and does neither teach nor suggest that the relevant feature is the total content of only AgCl.

US 2019/0320923 A1 describes a bioelectrode, which contains an electrode film that contains supported AgCl supported on support particles. The document teaches that this electrode film makes it possible to reduce the total amount of silver as compared to the bioelectrode disclosed in Japanese Patent Application Publication, Tokukaihei, No. 5-95922, when both electrodes are to achieve the same total area of exposure of AgCl within the electrode film.

The object of the present disclosure is to provide a medical electrode comprising a sensor element having a sensor layer, in which the amount of Ag and AgCl has been tailored for a specific application period, i.e. wear time, to provide high quality and stable signals without or with reduced risk of baseline drift. Preferably, the medical electrode is a single use bioelectric element, which is suitable for use in medical electrode applicable in ECG, EMG or EEG applications.

In view of this object, a novel medical electrode comprising a sensor element having a sensor layer, which contains Ag and AgCl, is provided. The novel medical electrode is based on the surprising finding that the application time, i.e. maximum wear time before baseline drift could occur, of a medical electrode depends on the amount of AgCl readily available on the surface of the sensor layer. The total amount of AgCl throughout the depth of the sensor layer is less relevant; only the amount of AgCl, which is available at the surface of the sensor element and thereby capable of taking part in the electrochemical reaction is relevant. This means that the thickness of the sensor layer is less important. The inventors have surprisingly found that by carefully selecting 1) the effective AgCl area density at the surface of the sensor layer and 2) the surface area of the sensor layer, sensor elements, and thereby medical electrodes, can be designed with specific application time or wear time and thereby markedly reducing the amount of silver waste.

In this way a medical electrode with tailored amount of AgCl at the surface of the sensor layer is provided. The tailored amount is obtained by experimentally determining the effective AgCl area density at the sensor layer surface and then design a specific sensor layer area when the maximum wear time of the medical electrode is known.

The medical electrode as disclosed herein comprises a sensor element. The sensor element comprises a sensor layer at the surface, which contains a certain amount of silver (Ag) and silver chloride (AgCl). Typically, the amount of Ag and AgCl varies from one type of medical electrode to another and depends, among others, on how the sensor layer has been applied to the sensor element, the amounts of Ag and AgCl, respectively, in the ink or coat, which is applied to the surface of the sensor element and the specific method used to apply the ink or coat.

As mentioned above, the inventors of the present disclosure have surprisingly found that it is the amount of AgCl available at the surface of the sensor element, which is important when designing a medical electrode having a specific wear time. Typically, prior art sensor elements include an excess amount of Ag to ensure high electrical conductivity and lower amounts of the less conductive AgCl. However, since equal amounts of Ag and AgCl participate in the electrochemical detection of biopotentials signals, AgCl is typically the limiting factor. The focus of the present disclosure is the amount of AgCl. When designing the novel medical electrode as disclosed herein, the effective AgCl area density is experimentally determined as described in Examples 1, 2 and 3. Next, the surface area of the medical electrode's sensor layer is calculated using the equation as disclosed in claim 1. The surface area depends on the wear time aimed at. Hence, the disclosure herein makes it possible to design a medical electrode with specified wear time with minimum consumption of valuable Ag and AgCl.

Sensor element

The medical electrode comprises a sensor element and the sensor element comprises a sensor layer, which comprises silver (Ag) and silver chloride (AgCl). The sensor layer is in contact with the ion conductive layer, which comprises the chloride ions. At the interface between the sensor layer and the ion conductive layer the electrochemical reaction (see formula (1)) above) takes place. Due to this electrochemical reaction, electrons are produced and then transmitted to a monitor where the signal is processed and analyzed.

The amount of available Ag and AgCl on the sensor surface to participate in the electrochemical reaction depends on the chemical and physical composition of the conductive ink or coating composition such as the total silver content, particle size and shape and Ag:AgCl ratio. The application process when applying the Ag/AgCl sensor layer is also expected to influence the available amount of AgCl since it influences the surface topography and porosity. The relationship between available AgCl, sensor area and wear time can be defined by the effective AgCl area density. The method for determining the effective AgCl area density is described in Examples 1-3.

The effective AgCl area density is defined by the equation:

$$\rho_{A,AgCl} = C \cdot t_{app} \cdot \frac{1}{A_{sensor}}$$

where

- $\rho_{A,AgCl}$ is the effective AgCl area density at the surface of the sensor layer in mg/mm$^2$,
- C is a constant of $2.97*10^{-10}$ g/s,
- t is the wear time in seconds, and
- $A_{sensor}$ is the surface area of the sensor in mm$^2$.

The minimum effective AgCl area density is easily determined using the above equation when the sensor surface area and the wear time is specified. Table 1 below lists the minimum $\rho_{A,AgCl}$ of a medical electrode with a sensor surface area in the range of 10 to 100 mm$^2$ and designed to have a wear time of 1 to 10 days.

Table 1: Minimum $\rho_{A,AgCl}$ ($10^{-2}$ mg/mm$^2$) as a function of sensor surface area (mm$^2$) and wear time (days)

| | | Wear time (days) | | | | | | | | | |
|---|---|---|---|---|---|---|---|---|---|---|---|
| | | 1 | 2 | 3 | 4 | 5 | 6 | 7 | 8 | 9 | 10 |
| Sensor surface area (mm$^2$) | 10 | 0.257 | 0.513 | 0.770 | 1.027 | 1.284 | 1.540 | 1.797 | 2.054 | 2.310 | 2.567 |
| | 20 | 0.128 | 0.257 | 0.385 | 0.513 | 0.642 | 0.770 | 0.898 | 1.026 | 1.155 | 1.283 |
| | 30 | 0.086 | 0.171 | 0.257 | 0.342 | 0.428 | 0.514 | 0.599 | 0.685 | 0.770 | 0.856 |
| | 40 | 0.064 | 0.128 | 0.193 | 0.257 | 0.321 | 0.385 | 0.449 | 0.514 | 0.578 | 0.642 |
| | 50 | 0.051 | 0.103 | 0.154 | 0.205 | 0.257 | 0.308 | 0.359 | 0.410 | 0.462 | 0.513 |
| | 60 | 0.043 | 0.086 | 0.128 | 0.171 | 0.214 | 0.257 | 0.300 | 0.342 | 0.385 | 0.428 |
| | 70 | 0.037 | 0.073 | 0.110 | 0.147 | 0.184 | 0.220 | 0.257 | 0.294 | 0.330 | 0.367 |
| | 80 | 0.032 | 0.064 | 0.096 | 0.128 | 0.161 | 0.193 | 0.225 | 0.257 | 0.289 | 0.321 |
| | 90 | 0.029 | 0.057 | 0.086 | 0.114 | 0.143 | 0.171 | 0.200 | 0.228 | 0.257 | 0.285 |
| | 100 | 0.026 | 0.051 | 0.077 | 0.103 | 0.129 | 0.154 | 0.180 | 0.206 | 0.231 | 0.257 |

In the same way, the sensor surface area can be determined using the above equation when the effective AgCl area density and the wear time is specified. Table 2 below lists the minimum sensor surface area of a medical electrode having an effective AgCl area density in the range of 0.01 to 3.5 ($10^{-2}$ mg/mm$^2$) and designed to have a wear time of 1 to 10 days.

Table 2: Minimum sensor surface area (mm$^2$) as a function of $\rho_{A,AgCl}$ ($10^{-2}$ mg/mm$^2$) and wear time (days)

| | | Wear time (days) | | | | | | | | | |
|---|---|---|---|---|---|---|---|---|---|---|---|
| | | 1 | 2 | 3 | 4 | 5 | 6 | 7 | 8 | 9 | 10 |
| $\rho_{A,AgCl}$ ($10^{-2}$ mg/mm$^2$) | 0.01 | 257 | 513 | 770 | 1027 | 1283 | 1540 | 1797 | 2053 | 2310 | 2567 |
| | 0.1 | 25.7 | 51.3 | 77.0 | 102.7 | 128.3 | 154.0 | 179.7 | 205.3 | 231.0 | 256.7 |
| | 0.2 | 12.8 | 25.7 | 38.5 | 51.3 | 64.2 | 77.0 | 89.8 | 102.7 | 115.5 | 128.3 |
| | 0.5 | 5.1 | 10.3 | 15.4 | 20.5 | 25.7 | 30.8 | 35.9 | 41.1 | 46.2 | 51.3 |
| | 1.0 | 2.6 | 5.1 | 7.7 | 10.3 | 12.8 | 15.4 | 18.0 | 20.5 | 23.1 | 25.7 |
| | 1.5 | 1.7 | 3.4 | 5.1 | 6.8 | 8.6 | 10.3 | 12.0 | 13.7 | 15.4 | 17.1 |
| | 2.0 | 1.3 | 2.6 | 3.9 | 5.1 | 6.4 | 7.7 | 9.0 | 10.3 | 11.6 | 12.8 |
| | 2.5 | 1.0 | 2.1 | 3.1 | 4.1 | 5.1 | 6.2 | 7.2 | 8.2 | 9.2 | 10.3 |
| | 3.0 | 0.9 | 1.7 | 2.6 | 3.4 | 4.3 | 5.1 | 6.0 | 6.8 | 7.7 | 8.6 |
| | 3.5 | 0.7 | 1.5 | 2.2 | 2.9 | 3.7 | 4.4 | 5.1 | 5.9 | 6.6 | 7.3 |

The surface area of the sensor layer is typically at least 10 mm$^2$, or at least 100 mm$^2$ or at least 2000 mm$^2$, and/or the wear time is typically in the range of 5 minutes to 10 days. Electrodes with sensor areas smaller than 2000 mm$^2$ are relevant for most ECG, EEG, and EMG sensor designs, while electrodes with sensor areas greater than 2000 mm$^2$ typically are used as ground electrodes particularly in EMG applications.

The medical electrode may be adapted to have a wear time between 5 minutes and 10 days. A skilled person easily realizes that the amount of available AgCl in the sensor layer, i.e. the amount of AgCl which is available at the surface of the sensor layer, must be much less in medical electrodes, which are designed to perform for very short times, such as 5 minutes, as compared to medical electrodes designed for long term applications, such as up to 10 days. Therefore, the amount of silver and AgCl waste can be significantly reduced by first experimentally determining the effective AgCl area density as described in Examples 1-3 and then designing an appropriate sensor surface area to suit the particular wear time. As mentioned above, it is believed that the effective AgCl area density depends on the ink or the coating composition, the method of applying the ink or coat and the morphology of particles in the ink or coat.

Disclosed herein is a medical electrode with a sensor layer surface area of at most 100 mm² and having an effective AgCl area density of:

- at least 0.00026 mg/mm² when the wear time is set to 1 day, or
- at least 0.00051 mg/mm² when the wear time is set to 2 days, or
- at least 0.00077 mg/mm² when the wear time is set to 3 days, or
- at least 0.00103 mg/mm² when the wear time is set to 4 days, or
- at least 0.00129 mg/mm² when the wear time is set to 5 days, or
- at least 0.00154 mg/mm² when the wear time is set to 6 days, or
- at least 0.00180 mg/mm² when the wear time is set to 7 days, or
- at least 0.00206 mg/mm² when the wear time is set to 8 days, or
- at least 0.00231 mg/mm² when the wear time is set to 9 days, or
- at least 0.00257 mg/mm² when the wear time is set to 10 days.

Disclosed herein is also a medical electrode with a sensor layer surface area of at most 2000 mm² and having an effective AgCl area density of:

- at least 0.000013 mg/mm² when the wear time is set to 1 day, or
- at least 0.000026 mg/mm² when the wear time is set to 2 days, or
- at least 0.000039 mg/mm² when the wear time is set to 3 days, or
- at least 0.000052 mg/mm² when the wear time is set to 4 days, or
- at least 0.000065 mg/mm² when the wear time is set to 5 days, or
- at least 0.000078 mg/mm² when the wear time is set to 6 days, or
- at least 0.000091 mg/mm² when the wear time is set to 7 days, or
- at least 0.000104 mg/mm² when the wear time is set to 8 days, or
- at least 0.000117 mg/mm² when the wear time is set to 9 days, or
- at least 0.000130 mg/mm² when the wear time is set to 10 days.

The sensor element may be a printed sensor element or a sensor element with a coating comprising silver (Ag) and silver chloride (AgCl).

For example, the sensor element is a printed sensor where the Ag/AgCl sensor layer is printed on an electrically conductive layer. The electrically conductive layer may be a conductive substrate layer or a non-conductive substrate layer with a conductive ink printed onto the non-conductive substrate. An exemplary example of a printed sensor element is described in GB 2341104 B, where an electrically conductive layer is printed on a substrate and the Ag/AgCl layer is printed on the electrically conductive layer.

The substrate layer is typically a polymer film. Many polymer films are suitable as a substrate. Examples of substrates are films made of polyester, for example polyethylene terephthalate (PET), polycarbonate (PC), thermoplastic polyurethane (TPU), polyimide (PI), polyethylene (PE) or polypropylene (PP) or any mixture or blend thereof. The substrate layer may also be formed as a laminate comprising at least one layer made of polyethylene terephthalate (PET), polycarbonate (PC), thermoplastic polyurethane (TPU), polyimide (PI), polyethylene (PE) or polypropylene (PP) or any mixture or blend thereof.

The conductive substrate is typically formed by incorporating conductive particles into a non-conductive substrate layer. A skilled person would know how to select conductive particles. Typically, the conductive particles are selected from the group consisting of carbon, such as graphite particles, flakes and/or fibers. Alternatively or additionally, the non-conductive substrate may contain one or more metal particle(s), flake(s) or fiber(s). Suitable metals include tin, silver, gold, copper, platinum, palladium and alloys or mixtures of particles made of any one of the above-mentioned metals.

The non-conductive substrate does not contain any conductive particles. Instead a conductive ink is coated, e.g. by being printed, on the surface of the non-conductive substrate. The conductive ink comprises conductive particles. Such conductive particles are known in the art. Typically, the conductive particles are selected from the group consisting of carbon, such as graphite particles, flakes and/or fibers. Alternatively or additionally, the ink may contain one or more metal particle(s), flake(s) or fiber(s). Suitable metals in a conductive ink include tin, silver, gold, copper, platinum, palladium and alloys or mixtures of particles made of any one of the above-mentioned metals. A particularly preferred ink contains silver, e.g. as silver particles.

The electrically conductive layer may be further designed to comprise a track, which extends from the sensor area to the position where a fitting is arranged for establishing electrical connection between the sensor area and the fitting where a cord or wire is attached or where wireless connection means are arranged.

The medical electrode may alternatively comprise a cup shaped conductive substrate having an inner surface and an outer surface, where the sensor layer is applied onto the inner surface of the cup shaped substrate. The sensor layer is typically applied either by spraying or coating a composition comprising silver and AgCl on the inner surface of the cup shaped substrate. Typically, the substrate is molded in plastic, such as for example polyethylene terephthalate (PET), polycarbonate (PC), thermoplastic polyurethane (TPU), polyimide (PI), polyethylene (PE) or polypropylene (PP). The substrate may be a non-conductive substrate with a conductive ink or coating applied on the inner surface of the non-conductive substrate, or the substrate may be a conductive substrate formed by incorporating conductive particles as discussed above.

It is noted that a sensor element may alternatively be formed on the skin side of a fitting, e.g. a stud, by coating the skin side part of the stud with an AgCl or an Ag/AgCl coating. The fitting or stud may then be aligned with the central opening in the skin adhesive, and the ion conductive layer, e.g. by attaching the fitting or stud to the device backing, e.g. as described further below.

Ion conductive layer

The medical electrode includes an ion conductive layer to ensure electrical contact between the skin and the sensor element, which picks up the biopotential from the person wearing the medical electrode. The ion conductive layer includes an electrolyte. The electrolyte must at least contain chloride ions, because the chloride ions participate in the electrochemical reaction with Ag and AgCl present in the sensor layer. The ion conductive layer converts the biopotential signal from ion polarization to electrons, which are picked up in the sensor element and transferred to the device for monitoring, displaying and/or recording the biopotential.

The ion conductive layer may be a paste, a crosslinked hydrogel, a viscous noncrosslinked hydrogel or a conductive adhesive. A conductive adhesive is a skin adhesive comprising an electrolyte whereby a separate ion conductive layer is avoided.

The electrolyte contains salts dissolved in the water content in the ion conductive layer. These salts are typically highly soluble chloride salts, such as sodium chloride (NaCl), potassium chloride (KCl) or mixtures thereof.

Commercially available examples of a cohesive (crosslinked) skin friendly electrolyte hydrogel is Axelgaard AmGel AG625 and Ludlow hydrogel PR00383. Viscous gel and paste can be obtained commercially as Signa Gel from Parker and Ten20 conductive paste from Weaver and Company.

Skin adhesive

The skin adhesive can be any kind of adhesive such as those already used today in medical electrodes. The skin adhesive may be an acrylic based adhesive designed for medical and/or surgical use, which includes a pressure sensitive adhesive (PSA), e.g. by selecting an acrylic adhesive from the pool of acrylic adhesives used in medical electrodes and/or for medical or surgical applications. Additionally or alternatively, the skin adhesive may be a silicone based adhesive designed for medical and/or surgical use. The skin adhesive may also be an ion conductive skin adhesive comprising chloride ions, for example a solid gel with adhesive effect, so that the adhesive act both as the skin adhesive layer and as the ion conductive layer.

The skin adhesive may be a water absorbent skin adhesive preferably comprising hydrocolloids. Hydrocolloid adhesives are typically rubber-based pressure sensitive adhesives, which comprises colloid particles suspended or embedded in the polymer matrix. Acrylic-based hydrocolloid adhesives are also commercial available. The hydrocolloid particles allow the adhesive to absorb fluid until they become saturated. Hydrocolloid adhesives have different water absorption capacities, as they will absorb fluid until they become saturated. When the hydrocolloid adhesive becomes saturated, it forms a soft, moist gel, but may then loose at least some of its adherence to the skin.

For long-term medical electrodes, a high water absorption capacity is preferred to ensure sufficient attachment to the skin throughout the wear period, and to ensure the skin to stay healthy. In such cases, the skin adhesive preferably has a total water absorption capacity of at least 200 mg water/$cm^2$ so that sufficient attachment throughout a wear time of at least 4 days is obtained.

The water absorption capacity, sometimes called total water absorption (the two terms are used interchangeably in this disclosure), is determined by the uptake of water per $cm^2$ over a 24 hour period where the hydrocolloid adhesive is immersed in an (isotonic) saline solution of 0.9% (by weight) sodium chloride (NaCl) in water.

Commercially available hydrocolloid skin adhesives, which typically are developed for use in wound dressings or in ostomy applications, may be used in the present disclosure. Suitable examples include those from Avery Dennison, such as those sold under the tradenames MED 5577A, MED 5094H, MED 2171H, MED 5541H, MED 5589H, MED 5583H, MED 5542H and MED 2190H.

The water absorbent adhesive may further comprise an occlusive backing on the upper side, i.e. on the side facing away from the skin. The occlusive backing may be a separate backing layer attached to the hydrocolloid skin adhesive layer. Alternatively or additionally, the backing on the water absorbent skin adhesive layer may be composed of the electrode backing, which also covers the central area of the electrode with the sensor element and the ion conductive layer. Examples of suitable device backings and/or backings on the water absorbent skin adhesives are films of polyethylene terephthalate (PET), polyethylene (PE), polypropylene (PP), ethylene vinyl acetate (EVA), which is a copolymer of ethylene and vinyl acetate, polyvinylidene dichloride (PVDC) or any blend or mixture thereof. The backing may also be a laminate comprising one or more layers, where at least one layer is made of any one of the above-mentioned polymers. The occlusive backing for the hydrocolloid skin adhesive layer may be covered with a woven or non-woven fibrous layer, e.g. of polyethylene terephthalate (PET), polyethylene (PE), polypropylene (PP) or any blend or mixture thereof, to provide a soft surface, which may cause less mechanical stress on the skin surfaces in contact with the upper side of the medical electrode.

The water absorbent skin adhesive layer may have chamfered/beveled face at the circumference to reduce any sharp edges, which could be caught by clothing, itch or scratch surround skin. The beveled edges are believed to reduce the risk of edge lifting.


Rim skin adhesive

The medical electrode may further comprise a rim skin adhesive extending beyond a circumference of the skin adhesive layer in order to reduce edge lifting. The rim skin adhesive is a different type of skin adhesive than the skin adhesive. Optionally, the rim skin adhesive overlaps at least part of the upper side of the skin adhesive. The rim skin adhesive may fully overlap the upper side of the skin adhesive by forming an opening in the rim skin adhesive and aligning this opening with the opening in the water absorbent skin adhesive layer about the alignment axis.

The rim skin adhesive may be an acrylic based adhesive designed for medical and/or surgical use, which includes a pressure sensitive adhesives (PSA), e.g. by selecting an acrylic adhesive from the pool of acrylic adhesives, which are commonly used as skin adhesives in medical electrodes and/or for medical or surgical applications. Additionally or alternatively, the rim skin adhesive may be a hydrocolloid adhesive, e.g. an acrylic skin adhesive comprising hydrocolloid particles, for example with beveled edges. The rim skin adhesive may be selected to have a more tacky property than the skin adhesive.

The rim skin adhesive may also comprise a backing. The backing may be of the same material as the backing on the skin adhesive and/or the device backing or a different type of material. The backing on the rim skin adhesive does not need to be occlusive. Examples of suitable separate backings on the rim skin adhesives are films of polyethylene terephthalate (PET), polyolefins, such as polyethylene (PE), low density PE (LDPE), polypropylene (PP) or polyurethane (PU) or any blend or mixture thereof. The backing may also be a laminate comprising one or more layers, where at least one layer is made of any one of the above-mentioned polymers. The backing on the rim skin adhesive layer may comprise a woven or non-woven fibrous layer, e.g. of polyethylene terephthalate (PET), polyethylene (PE), polypropylene (PP) or any mixture thereof, to provide a soft surface, which may cause less mechanical stress on the skin surfaces in contact with the upper side or the circumferential edge of the medical electrode.


Device backing

The medical electrode also comprises a device backing layer. The device backing layer covers at least the sensor element and the ion conductive layer. The device backing layer may further cover the skin adhesive layer either partly or entirely.

The device backing layer in medical electrodes designed for long wear times is preferably made of an occlusive material. The occlusive material prevents ingress of water into the adhesive if the medical electrode is worn while taking a shower, which otherwise will result in a potential loss of tackiness, i.e. adhesion to the skin is reduced. Ingress of water into the sensor area may also affect the signal quality in a negative way. For example, ingress of water into the ion conductive layer could lower ion concentration in the electrolyte gel and result in higher impedance.

For medical electrodes designed for short wear times, the backing need not to be of an occlusive material. The use of non-occlusive backing materials makes it possible to design fully breathable medical electrodes.

The device backing may be made of for example polyethylene terephthalate (PET), polyethylene (PE), polypropylene (PP), polyimide (PI), polyurethane (PU), thermoplastic polyurethane (TPU) or polycarbonate (PC), or it may be a blend or mixture thereof or a laminate comprising one or more layers, where at least one layer is made of any one of the above-mentioned polymers.

The device backing is attached to the skin adhesive and/or the rim skin adhesive to cover and seal the opening in at least the skin adhesive. Hereby a sealed compartment around the sensor element and the ion conductive layer is formed to eliminate any escape of water from the ion conductive layer as this could compromise the performance of the medical electrode by an increased impedance caused by a decrease in water activity in the ion conductive layer.

The thickness of the device backing layer must on the one hand be so thick that it protects the sensor element, but on the other hand it must not be so thick that it impedes the freedom of movement of the person wearing the medical electrode.

The device backing layer may e.g. be circular, oval or polygonal or more complex shaped, e.g. to correspond to the shape of the medical electrode.

The device backing may have teardrop shape whereby the broad part of the teardrop shape covers the sensor element and the ion conductive layer arranged in the opening in the skin adhesive. The fitting may then be arranged at a narrow end of the teardrop form to offset the connection of wires or cords from the sensor element's position.

The narrow end of the teardrop form does preferably not extend beyond the outer circumference of the skin adhesive layer and/or the rim skin adhesive. This will allow the patient's skin to be protected from mechanical impact, e.g. scratches, from the fitting, and/or the connector on the wire or cord.

Fittings

The medical electrode typically comprises fittings for connecting the sensor element to a wired or wireless connection to the device for monitoring, displaying and/or recording the biopotential signals. Such fittings are well-known to the skilled person, and may e.g. be formed as a wire, a snap, a socket for a banana plug, or a stud. The connector may, alternatively, be a part of the device backing layer of the medical electrode, which is formed as a tab onto which a (wired) clip may be attached, a conductor connected to the sensor element or the sensor element may be attached to the underside of the tab for establishing electrical contact between the sensor element and the wired clip.

In a wireless electrode, the fitting may comprise a connection to an electronic circuitry box arranged on the electrode or at a separate patch, which comprises wireless communication means, including, if necessary, means for transferring the analog signals from the medical electrode into digital signals. Additionally, a wireless medical electrode may comprise a plurality, e.g. 2, 3, 4, 5 or more separate sensor elements each arranged in a separate opening in the water absorbent skin adhesive layer and each provided with an ion conductive layer arranged in the opening as described above.

The medical electrode may further comprise a cord, which at its first end is in electrical connection with the sensor element and which at its second end is connectable to a device for monitoring, displaying and/or recording the biopotential signals, e.g. by means of a male or female connector.

The parts of the medical electrode are assembled, e.g. by gluing the parts together. Alternatively, the medical electrode can be assembled using hot melts, or by selecting a welding process which is suitable for use with the materials used, in particular materials used in the device backing and the backing layer(s) of the water absorbent adhesive and/or the rim skin adhesive. The welding methods may e.g. be ultrasonic welding, high frequency welding, laser welding, or hot press where applicable.

Release liner

Typically, the medical electrode further comprises a release liner to protect the skin side of the medical electrode during storage. The release liner is removed only just before use. A commercially available release liner may be used, e.g. a siliconized film or laminate comprising PET or PE.

A person skilled in the art will appreciate that any one or more of the abovediscussed aspects of this disclosure and embodiments thereof may be combined with any one or more of the other aspects or embodiments thereof.

It is noted that the present disclosure is not limited to use exclusively with ECG electrodes and electrocardiography. The suggested solution is equally applicable with all types of biomedical surface electrodes, including biomedical surface electrodes used in neurological studies, such as detecting bioelectrical brain signals, called EEG or electroencephalography, or detecting bioelectrical signals in skeletal muscle tissue, called EMG or electromyography.

The disclosure will now be explained in more detail below by means of examples of non-limiting embodiments with reference to the drawings.

Figure 1 shows a configuration of the medical electrode according to the disclosure without a rim adhesive.

Figure 2 a-c show variants of the medical electrode comprising rim skin adhesive with different overlap between the device backing, the rim skin adhesive and the water absorbent skin adhesive.

Figure 3 a-b show examples of sensor elements that can be used in the present disclosure.

Figure 4 a-g show different sizes and shapes of the device backing layer.

Figure 5 a-d show different types of fittings that may be used in the present disclosure.

Figure 6 a-c show an example of a cup electrode.

Figure 7 shows a typical ECG sensor attached to the skin of a human being and detecting a biopotential signal with an electrolyte gel and an Ag/AgCl electrode. The signal is transmitted to an ECG monitor.

Figure 8 shows ECG signals obtained simultaneously using a pair of ECG sensors with insufficient AgCl (top) and with sufficient AgCl (bottom), respectively. The placement of the two pairs of sensors is illustrated on the person to the left.

Figure 9 is a schematic illustration of chronopotentiometry for measuring the Ag or AgCl content available on the sensor surface. The sensor of interest is the working electrode, the counter electrode is much larger in area to the sensor. The current is induced between the working and counter electrode, while the potential is measured between the working and the well-defined reference electrode. All electrodes are connected to a potentiostat and immersed in gel containing ions.

Figure 10 shows the potential measured in chronopotentiometry as a function of time (a), charge (b) and AgCl amount (c). The asterisk in (c) represents a potential drop of 100 mV relative to the initial value.

Figure 11 shows results from chronopotentiometry measurements. The figure shows application time as a function of sensor area of different conductive Ag/AgCl inks. The minimum sensor area for application times of 5-8 days is indicated by the marked areas for their respective inks.

Figure 12 shows the effective AgCl area density as a function of sensor area for 1 day (a), 2 days (b), 5 days (c), 7 days (d), 10 days (e) and 14 days (f) of application time, respectively. The measured effective AgCl area densities for commercially available inks are plotted as horizontal lines for sensor areas where the effective AgCl area density is greater than the minimum area density for a given application time.

In the following, generally, similar elements of different embodiments have been designated by the same reference numerals when shown on different drawings. Thus, a feature discussed in relation to one drawing is not necessarily discussed in relation to all drawings.

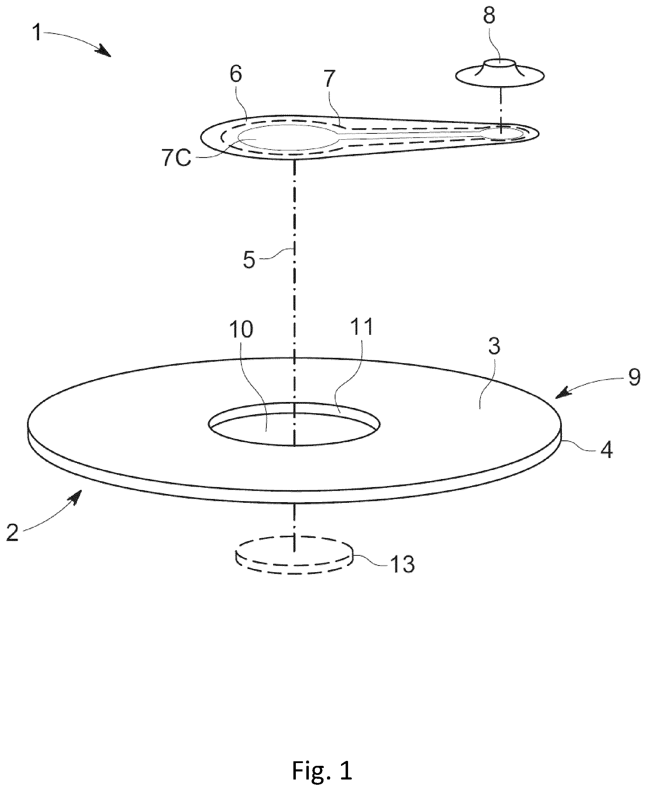

Fig. 1 shows a first example of the medical electrode 1 according to the present disclosure.

The medical electrode 1 comprises a skin adhesive layer 9 with an opening 10, which in the illustrated embodiment is central in the skin adhesive layer 9. The skin adhesive layer 9 has a skin side 2 intended for attachment to the skin of a patient and an upper side 3, which faces away from the patient's skin when the medical electrode 1 is applied to a patient. The skin adhesive layer 9 may be covered with an occlusive backing, which covers the upper side 3 of the skin adhesive layer 9. The occlusive backing may be a separate backing layer attached to the skin adhesive layer 9 and/or the backing on the skin adhesive layer 9 may be composed of a device backing layer 6, which also covers an area of the electrode with a sensor element 7 and an ion conductive layer 13 as discussed further below.

The skin adhesive layer 9 may have a chamfered/beveled face (not shown in figs.) at the outer circumference 4 to reduce any sharp edges, which could be caught by clothing, itch or scratch the patient, e.g. at the surrounding skin or skin which comes into contact with the upper side 3 of the skin adhesive layer 9. The beveled face of the edges is also believed to reduce the risk of edge lifting, i.e. when the adhesive slips the skin at the outer circumference 4 of skin adhesive layer 9.

In the drawings the medical electrode 1 is shown as a circular device. It is noted that the skilled person will immediately realize that a medical electrode does not need to be circular. Other shapes are possible, such as oval or elliptical devices or polygonal devices, e.g. square, rectangular hexagonal, octagonal etc. shapes are possible, e.g. with rounded corners. Also, more complex shapes of the medical electrode are possible, such as composed of a combination of a circle, oval or polygon formed integral with a tab like element having e.g. a rectangular or square shape, e.g. with rounded corners, with circular or oval shape. It is also noted that the opening 10 is not necessarily arranged centrally in the skin adhesive layer 9, as it may also be offset from the center of the skin adhesive layer 9 or of the electrode 1.

The skin adhesive layer 9 may have an opening 10, which is throughgoing and extends from the upper side to the skin side 2 of the layer.

The device backing layer 6 is attached to the upper side 3 of the skin adhesive layer 9 and covers the opening 10 in the skin adhesive layer 9. The device backing layer 6 is discussed in further detail below.

The sensor element 7 is arranged between the device backing layer 6 and the skin adhesive layer 9 as outlined in dotted lines in fig. 1. The sensor element may be a printed sensor element 7a as illustrated in figs. 1-3 and 5.

The sensor area 7b of the sensor element 7 may be aligned along an alignment axis 5 with the opening 10 in the skin adhesive layer 9 and the ion conductive layer 13.

The ion conductive layer 13 may fill the opening 10 entirely or partly, e.g. by leaving a gap of 0.5-1.5 mm between the inner circumference 11 of the skin adhesive layer 9 and the outer circumference of the ion conductive layer 13.

The ion conductive layer 13 is in direct contact with the sensor area 7b of the sensor element 7, i.e. the Ag/AgCl coated part of the printed sensor 7a.

It is noted that if a conductive adhesive is used as the skin adhesive 9, then the entire skin adhesive will be ion conductive, whereby the separate ion conductive layer 13 and the opening 10 in the skin adhesive layer may be omitted.

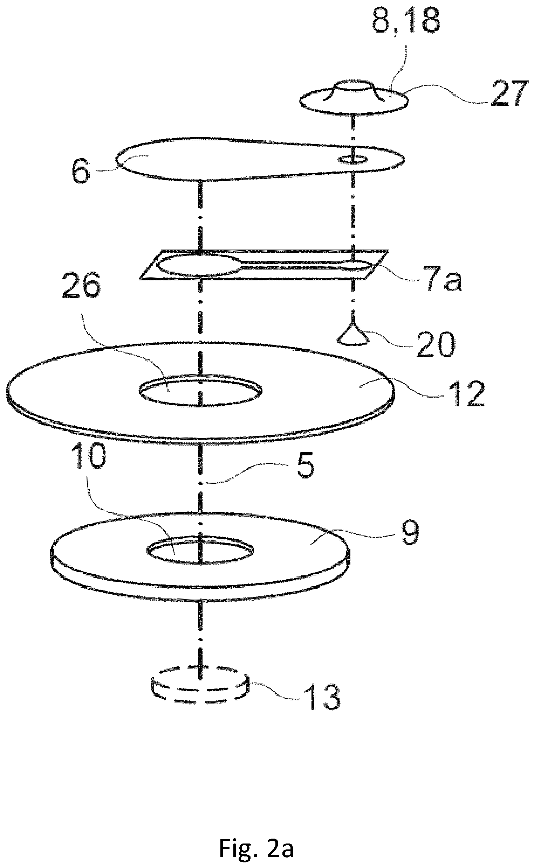

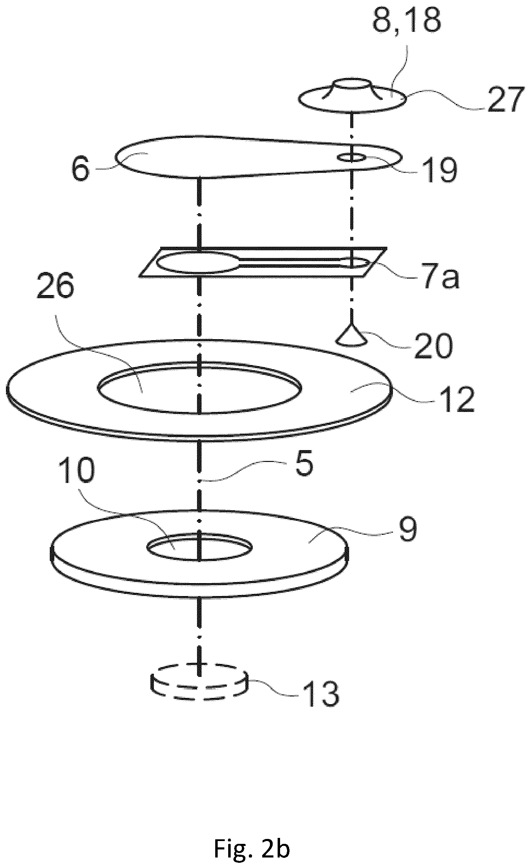

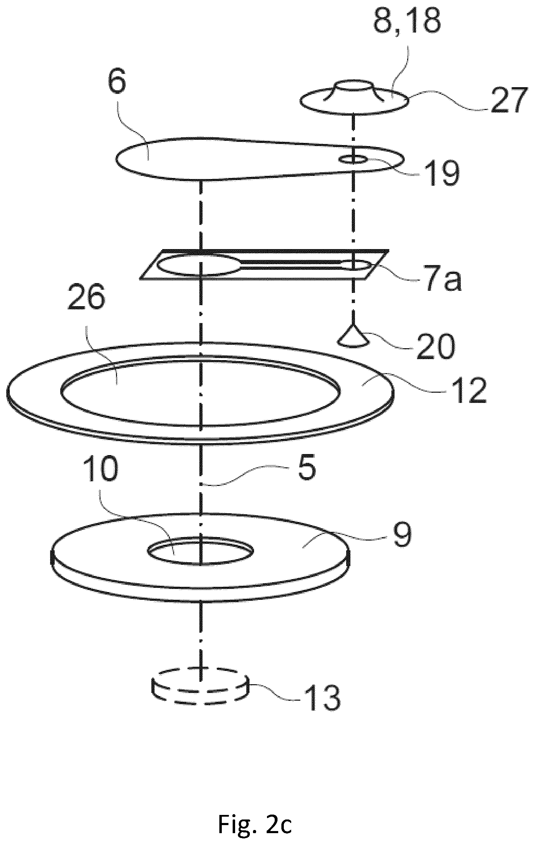

A fitting 8 is attached to the device backing layer 6 and arranged in electrical contact with the sensor element 7. In fig. 1, the fitting shown is a stud, which is typically a two-part stud (see also figs. 2-3), which is used to connect a wire or cord to the medical electrode to transfer bioelectrical signals to a monitor. Alternative fittings or connectors are discussed further below and may be used as alternative to the fitting shown in fig. 1.

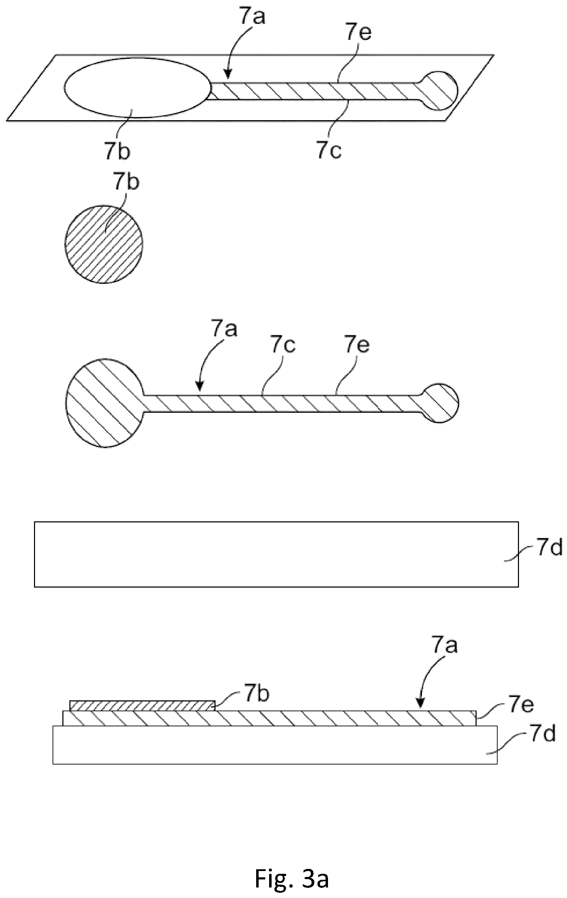

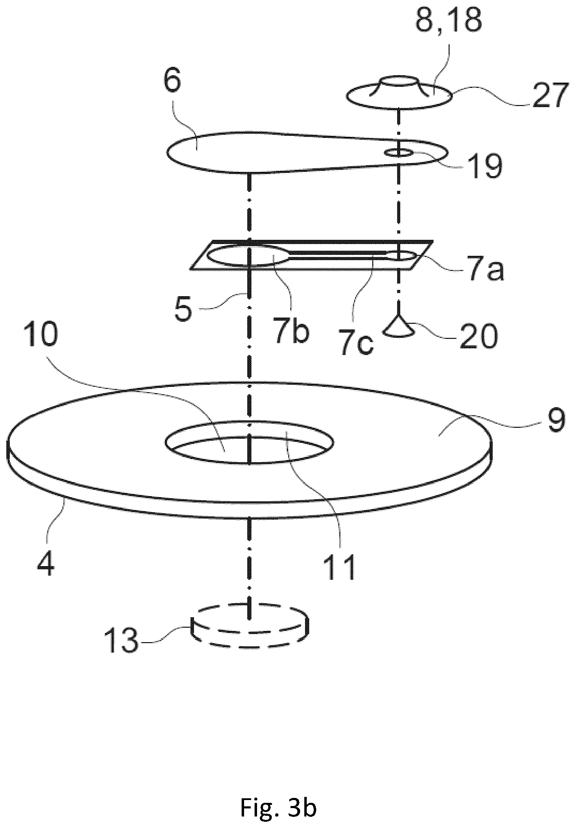

Fig. 3a illustrates a similar configuration where a printed sensor element 7a is shown. The printed sensor element may be a printed sensor element 7a formed on a substrate film 7d as described above. The printed sensor element 7a may be further designed to comprise a conductor track 7c, which extends from the sensor area 7b to the position where a fitting 8 is arranged. Thereby the conductor track 7c establishes electrical connection between the sensor area 7b and the fitting 8 where a cord or wire is attached or where wireless connection means are arranged. The printed sensor element 7a is arranged with the sensor area 7b as well as the electrically conductive layer forming the conductor track 7c directed towards the skin side 2 skin adhesive layer 9.

Fig. 2a-2c illustrates variants of the medical electrode 1, which further comprises a rim skin adhesive 12. The rim skin adhesive 12 is arranged to extend radially beyond a circumference of the skin adhesive layer 9. The rim skin adhesive 12 is preferred when it is considered necessary to reduce edge lifting. The rim skin adhesive 12 may overlap at least part of the upper side of the skin adhesive layer 9. In fig. 2a the rim skin adhesive 12 fully covers the upper side of the skin adhesive layer 9 whereby the opening 10 in the skin adhesive layer 9 and an opening 26 in the rim skin adhesive 12 have the same diameter and are aligned about the alignment axis 5.

In fig. 2b, the opening 26 in the rim skin adhesive 12 is relatively small compared to the outer circumference of the skin adhesive layer 9 and therefore, there is a substantial overlap between the rim skin adhesive 12 and the skin adhesive layer 9.

In fig. 2c, this overlap is minimal, such as in the range of 3-10 mm, allowing only for attaching the inner circumference of the rim skin adhesive 12 to the backing on the upper side 3 near the outer circumference of the skin adhesive layer 9.

It is noted that although figs. 2a-2c are drawn with a fitting 8 shown as a stud 18, any suitable fitting 8, e.g. as discussed below, may be used.

In variants of the medical electrode 1, the skin adhesive layer may be formed by a water absorbent skin adhesive. In such variants the water absorbent skin adhesive may absorb water from the ion conductive layer, in particular if the ion conductive layer has a high water activity or low viscosity, e.g. if a wet gel, is used. In such cases, it may be necessary to include a water barrier at or adjacent to the inner circumference 11 of the skin adhesive layer 9, i.e. between the ion conductive layer 13 and the skin adhesive layer 9 to protect the skin adhesive layer 9 from absorbing water from the ion conductive layer 13, at least during storage of the medical electrode 1. The water barrier may be a thin lacquer, a foam ring with closed cell structure or a compartment in the release liner. The barrier is not shown in the figures.

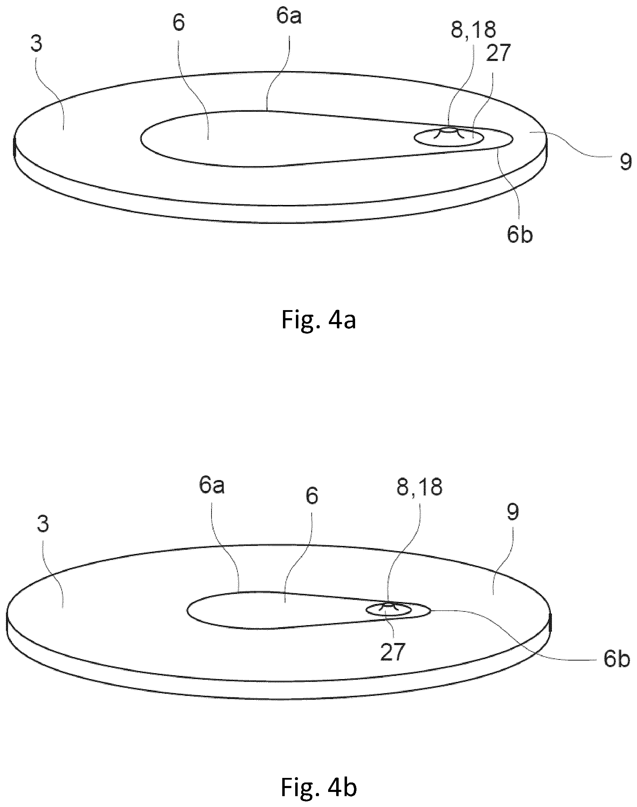

Figs 4a-4g illustrate different examples of shapes of a device backing layer 6 of the medical electrode 1. As already mentioned above, the device backing layer 6 covers at least the opening 10, sensor element 7 and the ion conductive layer 13. The device backing layer 6 may further cover the skin adhesive layer 9 either partly or entirely. The device backing layer 6 may further overlap with the inner circumference of the rim skin adhesive 12 where applicable. Figs 4a-4c illustrate different sizes of the device backing layer 6 on the medical electrode 1 according to the present disclosure, here exemplified by a medical electrode 1 with a skin adhesive layer 9.

As mentioned above, a preferred shape of the device backing layer 6 is the teardrop shape as shown in figs. 4a-4e. The teardrop shaped device backing layer 6 is arranged to cover the sensor element 7 (not seen in in fig. 4a-g), the ion conductive layer 13 (a gel layer) (not seen in in fig. 4a-g) and the opening 10 (not seen in in fig. 4a-g) in the skin adhesive layer 9 and/or the opening 26 in the rim skin adhesive 12. The broad part 6a of the teardrop shaped device backing layer 6 covers the opening 10 and a part of the upper side 3 of the skin adhesive layer 9 and/or the rim skin adhesive 12. The fitting 8 may then be arranged at a narrow part 6b of the teardrop form to offset the connection of wires or cords from the sensor element's 7 position.

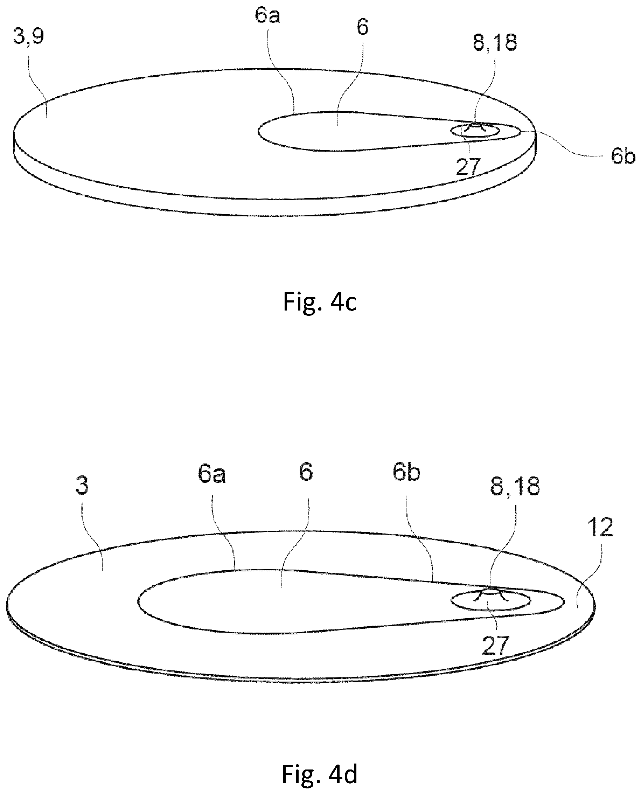

In the exemplified variants of the medical electrode 1 shown in fig. 4a and 4c, the broad part 6a of the teardrop shaped device backing layer 6 extends radially outside the opening 10 and onto the upper side 3 of the skin adhesive layer 9. The narrow part 6b allow attachment of a fitting (exemplified with a stud 18 in fig. 4a and 4c).

In fig. 4b, the broad part of the teardrop shaped device backing layer 6 is minimalized to extend 1-3 mm radially outside the opening 10 and onto the upper surface of the skin adhesive layer 9. The width and length of the narrow part 6b of the teardrop shaped device backing layer 6 is also minimized but still large enough to allow attachment of a fitting (exemplified with a stud 18 in fig. 4b).

The size and/or shape of the device backing layer 6 is dimensioned such that the fitting 8 does not cover the opening 10, neither partially nor entirely, in order to reduce the risk of mechanically induced artifacts caused by a pulling or moving wire/cord attached to the fitting 8.

The length of the device backing layer 6 is dimensioned such that the narrow part 8b which may carry a fitting 8 does not extend beyond the outer circumference 4 of the skin adhesive layer 9.

The teardrop shaped device backing layer's broad part 6a may be attached to the upper surface 2 of the skin adhesive layer 9 and/or the rim skin adhesive layer 12 to seal the opening 10.

The narrow part 6b of the device backing layer 6 is preferably not attached to the upper side 3 of the skin adhesive layer 9 to ease attachment of the wire or cord to the fitting 8 (exemplified with a stud 18 in figs 4a-4e). The narrow part 6b of the teardrop form does preferably not extend beyond the outer circumference of the skin adhesive layer 9 and/or the rim skin adhesive 12. This will allow the patient's skin to be protected from mechanical impact, e.g. scratches from edges of the narrow end part of the device backing layer or from the fitting and/or the connector on the wire or cord, which may be attached thereto.

The upper side 3 of the skin adhesive layer 9 exemplified in figs. 4a-c is not covered by a rim skin adhesive 12. The variants shown in figs 4a-c may however comprise a rim skin adhesive 12, e.g. covering some or all of the upper side 3 of the skin adhesive layer 9, e.g. as illustrated in figs. 2a-2c.

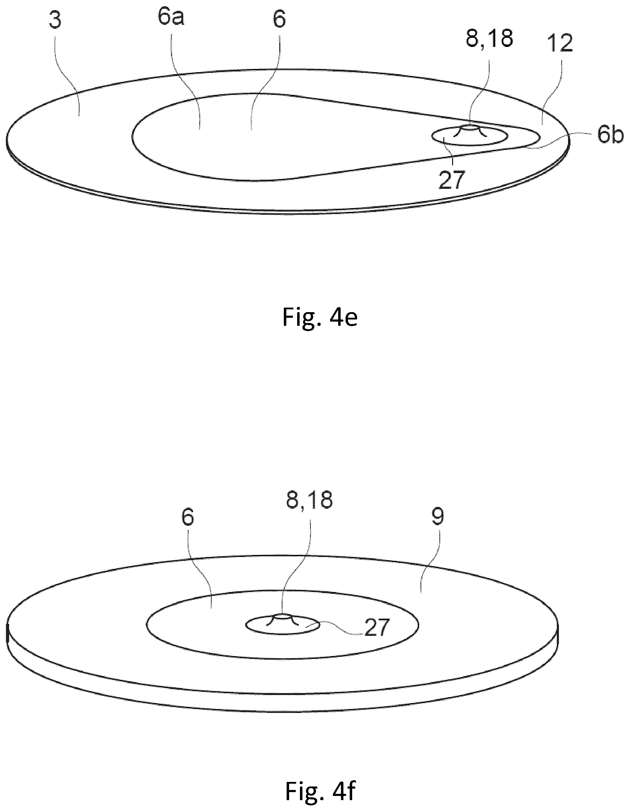

See also figs 4d-4e where the medical electrode 1 is shown with a teardrop shaped device backing layer 6 arranged on the upper side 3 of the skin adhesive layer 9 and covers the outer circumference of the skin adhesive layer 9 (not shown in fig. 4d-4e) and a part of an upper side of the rim skin adhesive 12. In figs. 4d-4e, the teardrop shaped device backing layer 6 also extends to cover a part of the upper side of the rim skin adhesive 12 but to a different degree in fig. 4d and 4e, respectively.

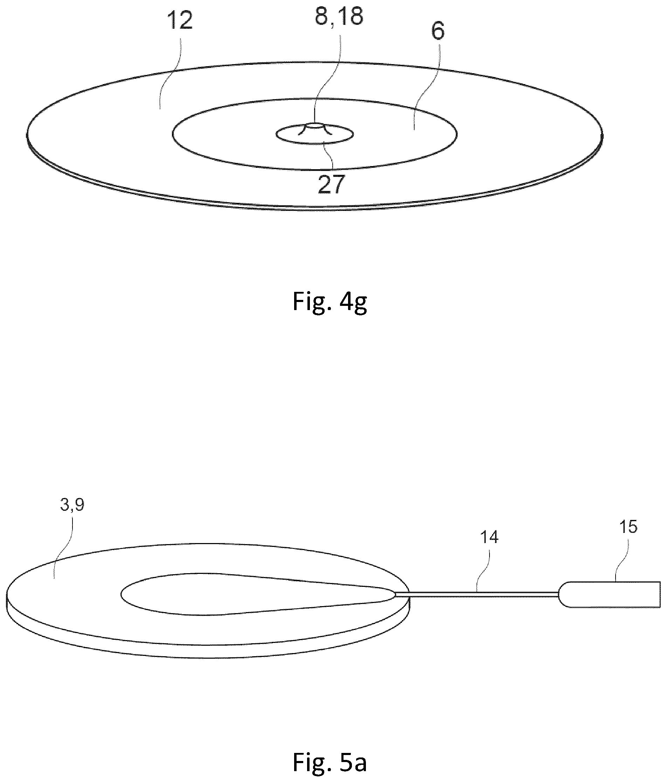

It is noted that a sensor element 7 may alternatively be formed on a skin side of a fitting, e.g. a stud 18, by coating the skin side of the stud with an AgCl or an Ag/AgCl coating to form a sensor layer 7b. The fitting 8 or stud 18 may then be aligned with the central or offset opening 10 in the skin adhesive layer 9, and the ion conductive layer 13, e.g. by attaching the fitting 8 or stud to the device backing layer 6, as shown in fig. 4f or 4g (sensor layer 7b on the skin side of stud 20 is not visible in figs. 4f-4g). As also shown in fig. 4f or 4g, this variant may comprise a device backing layer 6 with a geometry similar to the medical electrode's shape, here exemplified with a circular design, although other shapes are possible, e.g. as already discussed above. Alternatively, where an ion conductive adhesive is used as the skin adhesive layer 9, the opening 10 for arranging an ion conductive layer 13 is omitted. The fitting 8, stud 18,20 may then be aligned with the center of the medical electrode or the stud 18,20 may be offset from the center of the electrode, if so preferred. The area of the skin side of the stud 18 defines a maximum sensor area in this variant.

Typically, the thickness of the device backing layer 6 may be in the range of 25-100 $\mu$m.

Figs. 5a-5d exemplify a number of different types of fittings 8 or connectors, which are equally applicable in all exemplified variants of the medical electrode 1 discussed above. The fittings 8 or connectors 8 are all intended for connecting the sensor element to a wired or wireless connection to the device for monitoring, displaying and/or recording the biopotential signals.

The fitting shown in figs. 1-3 and figs. 4a-4g is a commonly used connector in the form of a stud 18. The stud 18 has a first part 27 arranged on the top side of the device backing layer 6. A hole 19 is punched in the device backing layer 6 and the stud's second part 20 is inserted into the hole and attached to the first part 27, e.g. by riveting and/or gluing.

In fig. 4f-4g the hole is punched centrally in the backing to arrange the first part 27 of the stud 18 in direct contact with the ion conductive layer 13. The fitting 8, in particular a stud 18, and where applicable the opening 10, may also be positioned offset relative to the center of the medical electrode 1.

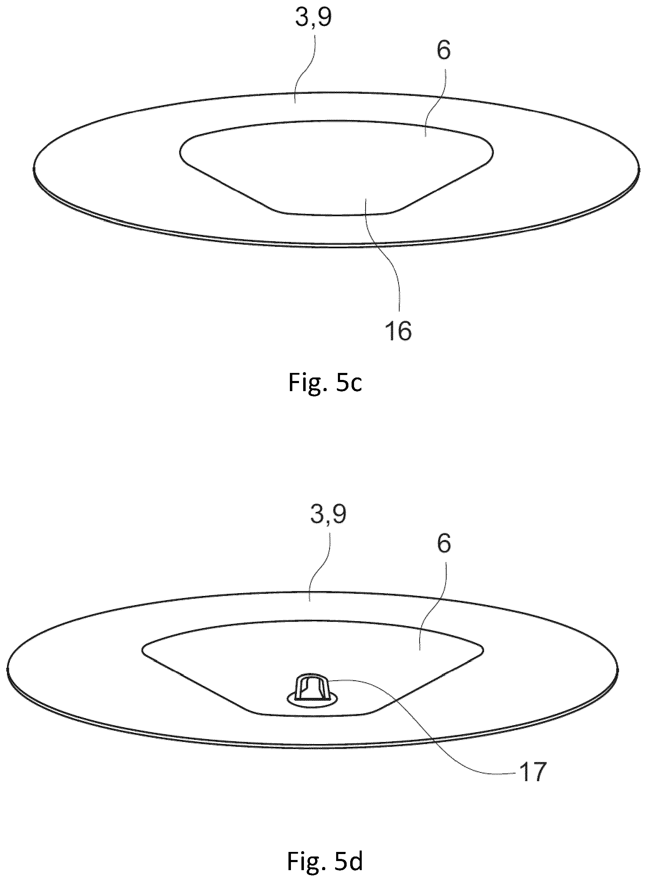

The fitting 8 may, as exemplified in fig. 5d comprise a socket 17 for connecting to a banana plug (not shown).

The fitting may, as exemplified in fig. 5c, be a part of the device backing layer of the medical electrode, which is formed as a tab 16 onto which a wired clip (not shown) may be attached. A conductor track 7c connected to the sensor element 7 or the sensor element itself may be attached to the underside of the tab 16 for establishing electrical contact between the sensor element and the wired clip.

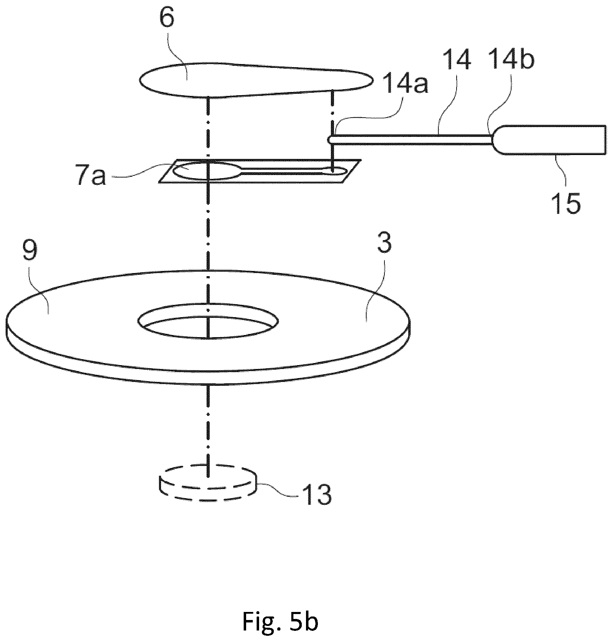

As shown in fig. 5a-5b, the medical electrode 1 may comprise a cord or wire 14, which at its first end 14a is attached in the medical electrode 1 in electrical connection with the sensor element 7. At the second end 14b, the wire or cord 14 is connectable to a device for monitoring, displaying and/or recording the biopotential signals, e.g. by means of a male or female connector 15. As exemplified in fig. 5b the sensor element 7 may be a printed sensor element.

Although the above description and the figures illustrate a medical electrode having an ion conductive layer 13 and at least one skin adhesive layer 9, it is noted that the electrode may also be formed with a conductive adhesive whereby a separate ion conductive layer is avoided.

The parts of the medical electrode are assembled, e.g. by gluing the parts together or by welding as discussed above.

Typically, the medical electrode 1 further comprises a release liner attached to the skin side 2 of the skin adhesive layer during storage. For clarity, the release liner is not shown in the drawings.

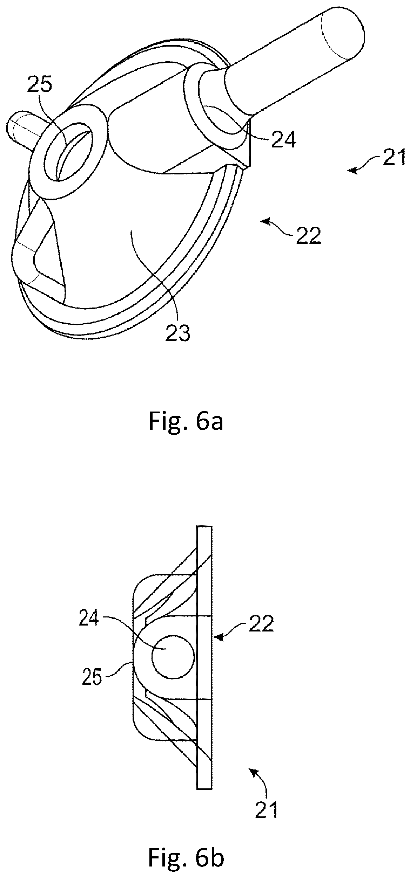

The medical electrode may also be formed as a cup electrode 21 as shown in Fig. 6. In this variant, the substrate is shaped as a cup having an inner surface 22 and an outer surface 23. The sensor layer is applied onto the inner surface 22 of the cup. The sensor area 7b may be applied for example by spraying an ink onto the inner surface 22 of the cup or by using a coating process. Typically, the substrate is molded in a plastic material. The substrate may be a non-conductive substrate with an electrically conductive coating, i.e. a conductive ink, coated onto the inner surface 22 of the non-conductive substrate of the cup, or it may be a conductive substrate formed by incorporating conductive particles into the plastic material, such as by mixing conductive particles as mentioned above into the molten plastic material mass prior to forming the cup electrode 21. The sensor area 7b may then be coated onto the electrically conductive coating layer or on the conductive substrate, respectively.

The cup electrode 21 is provided with a small hole 25 at the upper side of the cup. The hole 25 is facing away from the skin side of the cup. The hole 25 permits the ion conductive layer material, e.g. an ion conductive paste, such as Ten20 gel, to be filled into the cup and also convenient allows excess ion conductive paste to escape during cup positioning. In addition, the hole 25 permits refilling of paste into the cup, when necessary and/or insertion of needles, when necessary. The cup is provided with a tube stub 24 through which the wire or cord can run (wire not shown in fig. 6 a-c). The wire or cord is at its first end in electrical connection with the sensor area 7b, e.g. via a printed conductor 7e formed on the inner side 22 of the cup or by the cup being formed from a conductive substrate, e.g. inside the tube stub 24 arranged at extend laterally from the outer surface of the cup. At its second end the wire is connectable to a (not shown) device for monitoring, displaying and/or recording the biopotential signals, e.g. by means of a male or female connector (also not shown in figs.).

## EXAMPLES

### Example 1. Determination of total amount of AgCl readily available at the electrode surface

Chronopotentiometry can be used to measure an electrode's ability to maintain a stable half-cell potential when continuously exposed to a flow of charges as illustrated in Figure 9. A constant current is induced between the electrode of interest (working electrode) and a counter electrode with a potentiostat (PalmSens4, PalmSens BV, Netherlands). Both electrodes are Ag/AgCl electrodes. The area of the counter electrode is much larger than the working electrode to ensure that it will not be electrochemically limiting (> 15 cm$^2$). The constant current simulates the leakage current and shifts the electrochemical equilibrium at the working electrode surface. The current direction dictates the direction that the electrochemical reaction is shifted. To obtain reliable half-cell potential measurements, the surface potential of the working electrode is measured against a well-defined reference electrode (Ag/AgCl reference electrode, Metrohm, Denmark). In this measurement, the electrodes are immersed in an electrolyte with a sodium chloride concentration of 8 wt.%, the current is -100 $\mu$A (I = -100 $\mu$A), which decreases the test time compared to the much lower leakage current of 200 nA ($I_{leak}$ = 200 nA) according to the ANSI AAMI EC12 (2015) standard mentioned above. The increased current may cause the potential shift to occur earlier and is therefore considered to be a conservative test. For comparison of sensors with different areas, A, the current density, I/A, is kept constant. The tests were carried out at room temperature ($\approx$22°C).

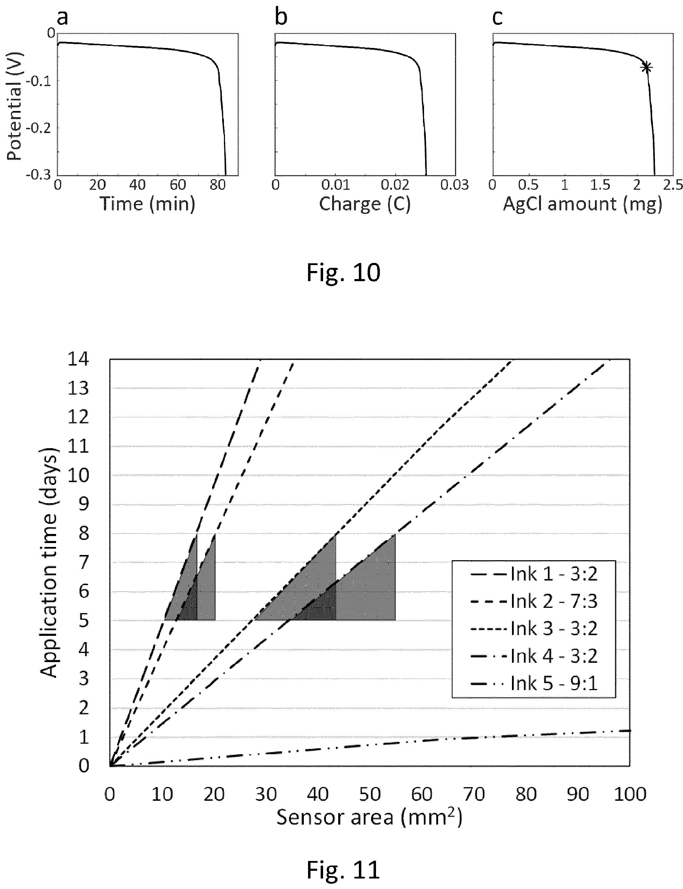

An example of data obtained by chronopotentiometry is presented in Figure 10. Figure 10a shows the potential as a function of time.

Figure 10b shows the potential as a function of charge exposed to the electrode, which can be determined using the equation:

$$Q = I \cdot t, \qquad\qquad (2)$$

where Q is the charge in coulomb I, I is the current in ampere (A = C/s), and t is the time (s). Assuming that all electrons introduced to the electrode through the induced current participate in the electrochemical reaction, the amount of AgCl can be estimated using the equation:

$$m_{AgCl}(t) = \frac{I \cdot t \cdot M_{w,AgCl}}{e^- \cdot N_A} \qquad\qquad (3)$$

where $m_{AgCl}$ (g) is the amount of AgCl available at the electrode surface, $M_{w,\,AgCl}$ (g/mol) is the molecular weight of AgCl, e$^-$ = 1.602 * 10$^{-19}$ C is the elementary charge, and $N_A$ = 6.022 * 10$^{23}$ mol$^{-1}$ is Avogadro's number.

Figure 10c shows the potential as a function of amount AgCl. In this figure, it is shown that the potential exhibits a plateau followed by a dramatic drop in potential. The drop in potential indicates that the electrode no longer has AgCl readily available at the surface to participate in the electrochemical reaction. The point of the drop is defined in ANSI AAMI EC12 (2015) as $V_{drop}$ = $V_0$ - 50 mV, where $V_0$ is the initial potential. The amount of AgCl at the potential drop, $m_{AgCl}$ ($t_{drop}$), indicated by the asterisk in Figure 10c, is estimated as the total amount of AgCl readily available at the electrode surface.

### Example 2. Determination of sensor application time

The total AgCl amount is correlated to the maximum sensor application time. By conversely assuming that the current density does not influence the potential drop, the maximum sensor application time, $t_{app}$, can be calculated for a given leakage current, $I_{leak}$, using the equation:

$$t_{app} = \frac{I}{I_{leak}} \, t_{drop} \qquad (4)$$

This equation gives a simple relationship between the maximum sensor application time and the time of potential drop, $t_{drop}$, in chronopotentiometry with current, I.

Chronopotentiometry measurements as described in Example 1 were performed using different screen-printed Ag/AgCl inks printed in different areas. The results are shown in Figure 11 as application time as a function of sensor area. The total silver content is unknown, but the ratio of silver and silver chloride (Ag:AgCl) is known.

The tested inks are listed below:

Ink 1 - 3:2 having ratio of silver to silver chloride of 3:2,
Ink 2 - 7:3 having ratio of silver to silver chloride of 7:3,
Ink 3 - 3:2 having ratio of silver to silver chloride of 3:2,
Ink 4 - 3:2 having ratio of silver to silver chloride of 3:2, and
Ink 5 - 9:1 having ratio of silver to silver chloride of 9:1.

The results presented in Figure 11 shows that Ink 1 - 3:2 yielded the longest application time, followed by the Ink 2 - 7:3 and then Ink 3 - 3:2, which also indicates the order of the absolute silver chloride amount present in these inks. Ink 2 - 7:3 has a shorter application time compared to Ink 1 - 3:2, which is in agreement with the lower Ag:AgCl ratio. Ink 1 - 3:2 and Ink 4 - 3:2 both with Ag:AgCl ratio of 3:2 proved to have different application times indicating different absolute silver and silver chloride contents. It is seen from Figure 11 that Ink 1 - 3:2 has a longer application time compared to Ink 4 - 3:2, which indicates a greater absolute silver and silver chloride content in Ink 1 - 3:2.

The minimum sensor area required for application times of 5-8 days are indicated by the area under their respective lines in Figure 11. It is seen that Ink 5 - 9:1 would require a sensor area much greater than 100 $mm^2$ to yield an application time of 5 days.

The application time is proportional with the amount of AgCl available on the sensor surface to interface with the chloride ions in the gel and thus actively participate in the electrochemical reaction. The amount of available AgCl depends on the chemical and physical composition of the conductive ink such as the total silver content, particle size and shape and Ag:AgCl ratio. The application process, here exemplified by printing, is also expected to influence the sensor performance since it influences the surface topography and porosity. The relationship between active AgCl, sensor area and application time can be defined by the effective AgCl area density, which is described below in Example 3, even without knowing the absolute content of silver and silver chloride in the inks or coatings used to establish the sensor area on the sensor element.

**Example 3. Determination of effective AgCl area density**

The amount of AgCl ($m_{AgCl}$), which is available to participate in the electrochemical reaction during the application time, can also be described using equation (3) above:

$$m_{AgCl} = \frac{I_{leak} M_{w,AgCl}}{e^- \cdot N_A} \cdot t_{app} \qquad (5)$$

which shows a linear relationship between the amount of AgCl and application time. For simplicity, equation (5) can be written as:

$$m_{AgCl} = C \cdot t_{app}, \qquad (6)$$

where $C = \frac{I_{leak} M_{w,AgCl}}{e^- \cdot N_A}$ is a constant.

The effective AgCl area density $\rho_{A,AgCl}$ ($mg/mm^2$) represents the amount of AgCl per area readily available to participate in the electrochemical reaction. The effective area density is constant and depends on the ink formulation and the printing process. The area density is defined as:

$$\rho_{A,AgCl} = \frac{m_{AgCl}}{A_{sensor}} \qquad\qquad (7)$$

The application time, $t_{app}$, can be can described by combining equations (6) and (7):

$$t_{app} = C^{-1} \cdot \rho_{A,AgCl} \cdot A_{sensor} \qquad\qquad (8)$$

To determine the effective AgCl area density, the derivative of the application time with respect to the sensor is first obtained:

$$\frac{dt_{app}}{dA_{sensor}} = C^{-1} \cdot \rho_{A,AgCl} \qquad\qquad (9)$$

and then equation (9) is rearranged to yield the effective AgCl area density:

$$\rho_{A,AgCl} = \frac{dt_{app}}{dA_{sensor}} \cdot C, \qquad\qquad (10)$$

where $\frac{dt_{app}}{dA_{sensor}}$ represents the slope of the plots in Figure 11. The effective AgCl area density is determined for the inks using equation (10) and the results are listed in Table 3.

Table 3: AgCl area density for the tested inks.

| Ink Ag : AgCl | $\rho_{A,AgCl}$ ($10^{-2}$ mg/mm$^2$) |
|---|---|
| Ink-1 - 3:2 | 1.29 |
| Ink-2 - 7:3 | 1.10 |
| Ink-3 - 3:2 | 0.49 |
| Ink-4 - 3:2 | 0.38 |
| Ink-5 - 9:1 | 0.07 |

Equation (8) can be rearranged to:

$$\rho_{A,AgCl} = C \cdot t_{app} \cdot \frac{1}{A_{sensor}} \qquad\qquad (11)$$

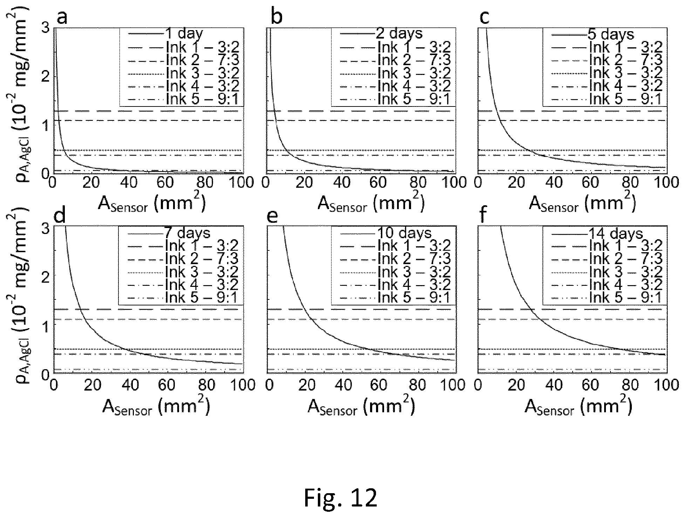

Equation (11) can be used to model the minimum required AgCl area density for a given application time and sensor area. The AgCl area density is plotted as a function of sensor area for 1, 2, 5, 7, 10 and 14 days in Figure 12. The area density for each ink is plotted in all sub figures.

It is clearly seen from Figure 12 that the minimum AgCl area density, marked by the solid lines, is proportional to the application time and inversely proportional to the sensor area. Figure 12 shows that a functional long-term sensor can be achieved by using an ink with a higher AgCl area density or printing a larger sensor surface area. For example, sensors used for 7 days (Figure 12(d)) can be made using Ink 1 -3:2 or Ink 2 - 7:3 with areas of approximately 20 mm$^2$ or Ink 3 - 3:2 or Ink 4 - 3:2 with areas of approximately 60 mm$^2$, while Ink 5 - 9:1 would not be feasible to achieve the desired application time. As an example of a sensor with sensor area of 50 mm$^2$, corresponding to a simple electrode with a stud coated with Ag/AgCl paste, the effective AgCl area density must be at least 0.0036 mg/mm$^2$ to achieve an application time of 7 days. For a medical electrode with an effective AgCl area density of 0.0013 mg/mm$^2$ and design for an application time of 5 says, the sensor area must be at least 100 mm$^2$.

List of reference numbers used in the drawings:

1. Medical electrode
2. Skin side of skin adhesive layer
3. Upper side of skin adhesive layer
4. Outer circumference of skin adhesive layer
5. Alignment axis
6. Device backing layer

    a. Broad part
    b. Narrow part

7. Sensor element

    a. Printed sensor element
    b. Sensor area
    c. Conductor track
    d. Substrate film
    e. Printed conductor

8. Fitting (connector)
9. Skin adhesive layer
10. Opening in skin adhesive layer
11. Inner circumference of skin adhesive layer
12. Rim skin adhesive
13. Ion conductive layer (electrolyte)
14. Wire, cord

    a. 1st end
    b. 2nd end

15. Male or female connector
16. Tab
17. Socket for banana plug
18. Stud
19. Hole in device backing layer
20. Second part of stud
21. Cup electrode
22. Inner surface
23. Outer surface
24. Tube stub
25. Hole
26. Opening of rim skin adhesive
27. First part of stud

**Claims**

**1.** A medical electrode for biopotential monitoring and/or recording of biopotential signals when attached to the skin of a human or animal, said medical electrode comprising

    - a sensor element comprising a sensor layer, and
    - an ion conductive layer in contact with the skin side of the sensor layer in the sensor element, said ion conductive layer comprising chloride ions,
    wherein the sensor layer comprises silver (Ag) and silver chloride (AgCl) and
    where the effective AgCl area density, $\rho_{A,AgCl}$, at the surface of the sensor layer is determined by the equation:

$$\rho_{A,AgCl} = C \cdot t_{app} \cdot \frac{1}{A_{sensor}}$$

where

- $\rho_{A,AgCl}$ is the effective AgCl area density at the surface of the sensor layer in mg/mm$^2$,
- C is a constant of $2.97*10^{-10}$ g/s,
- t is the wear time in seconds, and
- $A_{sensor}$ is the surface area of the sensor layer in mm$^2$, and

where the surface area of the sensor layer is at most 100 mm$^2$ and the effective AgCl area density is

- at least 0.00026 mg/mm$^2$ when the wear time is set to 1 day, or
- at least 0.00051 mg/mm$^2$ when the wear time is set to 2 days, or
- at least 0.00077 mg/mm$^2$ when the wear time is set to 3 days, or
- at least 0.00103 mg/mm$^2$ when the wear time is set to 4 days, or
- at least 0.00129 mg/mm$^2$ when the wear time is set to 5 days, or
- at least 0.00154 mg/mm$^2$ when the wear time is set to 6 days, or
- at least 0.00180 mg/mm$^2$ when the wear time is set to 7 days, or
- at least 0.00206 mg/mm$^2$ when the wear time is set to 8 days, or
- at least 0.00231 mg/mm$^2$ when the wear time is set to 9 days, or
- at least 0.00257 mg/mm$^2$ when the wear time is set to 10 days

or
where the surface area of the sensor layer is at most 2000 mm$^2$ and the effective AgCl area density is

- at least 0.000013 mg/mm$^2$ when the wear time is set to 1 day, or
- at least 0.000026 mg/mm$^2$ when the wear time is set to 2 days, or
- at least 0.000039 mg/mm$^2$ when the wear time is set to 3 days, or
- at least 0.000052 mg/mm$^2$ when the wear time is set to 4 days, or
- at least 0.000065 mg/mm$^2$ when the wear time is set to 5 days, or
- at least 0.000078 mg/mm$^2$ when the wear time is set to 6 days, or
- at least 0.000091 mg/mm$^2$ when the wear time is set to 7 days, or
- at least 0.000104 mg/mm$^2$ when the wear time is set to 8 days, or
- at least 0.000117 mg/mm$^2$ when the wear time is set to 9 days, or
- at least 0.000130 mg/mm$^2$ when the wear time is set to 10 days.

2. The medical electrode according to claim 1, wherein the surface area of the sensor layer is at least 10 mm$^2$, or at least 100 mm$^2$ or at least 2000 mm$^2$, and/or the wear time is in the range of 5 minutes to 10 days.

3. The medical electrode according to claim 1 or 2, wherein the medical electrode is adapted to a wear time of at least 5 minutes, such as at least 1 day or at least 4 days or at least 7 days or at least 10 days.

4. The medical electrode according to any one of the preceding claims, wherein the ion conductive layer is a paste, a cross-linked hydrogel, a viscous non-cross-linked hydrogel or an ion conductive adhesive.

5. The medical electrode according to any one of the preceding claims, wherein the sensor element is a printed sensor element comprising an electrically conductive layer in addition to the sensor layer, said electrically conductive layer is either a conductive substrate layer or a non-conductive substrate with a conductive ink layer coated, such as printed, onto the non-conductive substrate.

6. The medical electrode according to claim 5, wherein the substrate layer is made of polyethylene terephthalate (PET), polycarbonate (PC), thermoplastic polyurethane (TPU), polyimide (PI), polyethylene (PE) or polypropylene (PP) or any mixture or blend thereof, or the substrate layer is formed as a laminate comprising at least one layer made of polyethylene terephthalate (PET), polycarbonate (PC), thermoplastic polyurethane (TPU), polyimide (PI), polyethylene (PE) or polypropylene (PP) or any mixture or blend thereof.

7. The medical electrode according to claim 5 or 6, wherein the conductive substrate is formed by incorporating conductive particles into a non-conductive substrate layer, where said conductive particles is selected from the group consisting of carbon, such as graphite particles, flakes and/or fibers, or the conductive substrate may contain one or more particles, flakes and/or fibers of metal, such as tin, silver, gold, copper, platinum, palladium or alloys or mixtures thereof.

8. The medical electrode according to claim 5 or 6, wherein the conductive ink comprises conductive particles, where said conductive particles is selected from the group consisting of carbon, such as graphite particles, flakes and/or fibers, or the ink may contain one or more particles, flakes and/or fibers of metal, such as tin, silver, gold, copper, platinum, palladium or alloys or mixtures thereof.

9. The medical electrode according to any one of the preceding claims, wherein said medical electrode further comprises

   - a device backing, and
   - a skin adhesive having an upper side and a skin side adapted to attach to the skin of the human or animal.

10. The medical electrode according to any one of the preceding claims, wherein the medical electrode comprises a cup shaped conductive substrate having an inner surface and an outer surface, and where the sensor layer is applied onto the inner surface of the cup shaped substrate.

11. The medical electrode according to claim 10, wherein the conductive substrate is made of a non-conductive substrate having a conductive ink applied onto the inner surface of the cup shaped substrate.

**Patentansprüche**

1. Medizinische Elektrode zum Überwachen eines Biopotentials und/oder Aufzeichnen von Biopotentialsignalen, wenn sie an der Haut eines Menschen oder Tieres befestigt ist, wobei die medizinische Elektrode umfasst:

   - ein Sensorelement, umfassend eine Sensorschicht, und
   - eine ionenleitende Schicht in Kontakt mit der Hautseite der Sensorschicht in dem Sensorelement, wobei die ionenleitende Schicht Chloridionen umfasst,
   wobei die Sensorschicht Silber (Ag) und Silberchlorid (AgCl) umfasst und wobei die effektive AgCl-Flächendichte $\rho_{A,AgCl}$ an der Oberfläche der Sensorschicht durch die folgende Gleichung bestimmt wird:

$$\rho_{A,AgCl} = C \cdot t_{app} \cdot \frac{1}{A_{Sensor}}$$

   wobei

   - $\rho_{A,AgCl}$ die effektive AgCl-Flächendichte an der Oberfläche der Sensorschicht in mg/mm$^2$ ist,
   - C eine Konstante von 2,97*10$^{-10}$ g/s ist,
   - t die Tragezeit in Sekunden ist und
   - $A_{sensor}$ die Oberfläche der Sensorschicht in mm$^2$ ist, und

   wobei die Oberfläche der Sensorschicht höchstens 100 mm$^2$ beträgt und die effektive AgCl-Flächendichte

   - mindestens 0,00026 mg/mm$^2$ ist, wenn die Tragedauer auf 1 Tag eingestellt ist, oder
   - mindestens 0,00051 mg/mm$^2$ ist, wenn die Tragedauer auf 2 Tage eingestellt ist, oder
   - mindestens 0,00077 mg/mm$^2$ist, wenn die Tragedauer auf 3 Tage eingestellt ist, oder
   - mindestens 0,00103 mg/mm$^2$ ist, wenn die Tragedauer auf 4 Tage eingestellt ist, oder
   - mindestens 0,00129 mg/mm$^2$ ist, wenn die Tragedauer auf 5 Tage eingestellt ist, oder
   - mindestens 0,00154 mg/mm$^2$ ist, wenn die Tragedauer auf 6 Tage eingestellt ist, oder
   - mindestens 0,00180 mg/mm$^2$ ist, wenn die Tragedauer auf 7 Tage eingestellt ist, oder
   - mindestens 0,00206 mg/mm$^2$ ist, wenn die Tragedauer auf 8 Tage eingestellt ist, oder
   - mindestens 0,00231 mg/mm$^2$ ist, wenn die Tragedauer auf 9 Tage eingestellt ist, oder
   - mindestens 0,00257 mg/mm$^2$ ist, wenn die Tragezeit auf 10 Tage eingestellt ist,

   oder
   wobei die Oberfläche der Sensorschicht höchstens 2000 mm$^2$ beträgt und die effektive AgCl-Flächendichte

- mindestens 0,000013 mg/mm$^2$ ist, wenn die Tragedauer auf 1 Tag eingestellt ist, oder
- mindestens 0,000026 mg/mm$^2$ ist, wenn die Tragedauer auf 2 Tage eingestellt ist, oder
- mindestens 0,000039 mg/mm$^2$ ist, wenn die Tragedauer auf 3 Tage eingestellt ist, oder
- mindestens 0,000052 mg/mm$^2$ ist, wenn die Tragedauer auf 4 Tage eingestellt ist, oder
- mindestens 0,000065 mg/mm$^2$ ist, wenn die Tragedauer auf 5 Tage eingestellt ist, oder
- mindestens 0,000078 mg/mm$^2$ ist, wenn die Tragedauer auf 6 Tage eingestellt ist, oder
- mindestens 0,000091 mg/mm$^2$ ist, wenn die Tragedauer auf 7 Tage eingestellt ist, oder
- mindestens 0,000104 mg/mm$^2$ ist, wenn die Tragedauer auf 8 Tage eingestellt ist, oder
- mindestens 0,000117 mg/mm$^2$ ist, wenn die Tragedauer auf 9 Tage eingestellt ist, oder
- mindestens 0,000130 mg/mm$^2$ ist, wenn die Tragedauer auf 10 Tage eingestellt ist.

2. Medizinische Elektrode nach Anspruch 1, wobei die Oberfläche der Sensorschicht mindestens 10 mm$^2$ oder mindestens 100 mm$^2$ oder mindestens 2000 mm$^2$beträgt und/oder die Tragedauer im Bereich von 5 Minuten bis 10 Tagen liegt.

3. Medizinische Elektrode nach Anspruch 1 oder 2, wobei die medizinische Elektrode für eine Tragedauer von mindestens 5 Minuten, beispielsweise mindestens 1 Tag oder mindestens 4 Tage oder mindestens 7 Tage oder mindestens 10 Tage geeignet ist.

4. Medizinische Elektrode nach einem der vorstehenden Ansprüche, wobei die ionenleitende Schicht eine Paste, ein vernetztes Hydrogel, ein viskoses, nicht vernetztes Hydrogel oder ein ionenleitender Klebstoff ist.

5. Medizinische Elektrode nach einem der vorstehenden Ansprüche, wobei das Sensorelement ein gedrucktes Sensorelement ist, das zusätzlich zur Sensorschicht eine elektrisch leitfähige Schicht umfasst, wobei die elektrisch leitfähige Schicht entweder eine leitfähige Substratschicht oder ein nicht leitfähiges Substrat mit einer leitfähigen Tintenschicht ist, die auf das nicht leitfähige Substrat aufgetragen, beispielsweise aufgedruckt, ist.

6. Medizinische Elektrode nach Anspruch 5, wobei die Substratschicht aus Polyethylenterephthalat (PET), Polycarbonat (PC), thermoplastischem Polyurethan (TPU), Polyimid (PI), Polyethylen (PE) oder Polypropylen (PP) oder einem beliebigen Gemisch oder einer beliebigen Vermengung davon besteht, oder die Substratschicht als Laminat ausgebildet ist, das mindestens eine Schicht aus Polyethylenterephthalat (PET), Polycarbonat (PC), thermoplastischem Polyurethan (TPU), Polyimid (PI), Polyethylen (PE) oder Polypropylen (PP) oder einem beliebigen Gemisch oder einer beliebigen Vermengung davon umfasst.

7. Medizinische Elektrode nach Anspruch 5 oder 6, wobei das leitfähige Substrat durch Einbringen leitfähiger Partikel in eine nicht leitfähige Substratschicht gebildet wird, wobei die leitfähigen Partikel aus der Gruppe ausgewählt sind, die aus Kohlenstoff, wie Graphitpartikeln, Flocken und/oder Fasern besteht, oder das leitfähige Substrat ein oder mehrere Partikel, Flocken und/oder Fasern aus Metall, wie Zinn, Silber, Gold, Kupfer, Platin, Palladium oder Legierungen oder Gemische davon enthalten kann.

8. Medizinische Elektrode nach Anspruch 5 oder 6, wobei die leitfähige Tinte leitfähige Partikel umfasst, wobei die leitfähigen Partikel aus der Gruppe ausgewählt sind, die aus Kohlenstoff, wie Graphitpartikeln, Flocken und/oder Fasern besteht, oder die Tinte ein oder mehrere Partikel, Flocken und/oder Fasern aus Metall, wie Zinn, Silber, Gold, Kupfer, Platin, Palladium oder Legierungen oder Gemische davon enthalten kann.

9. Medizinische Elektrode nach einem der vorstehenden Ansprüche, wobei die medizinische Elektrode weiter umfasst

- eine Vorrichtungssicherung und
- einen Hautkleber, der eine Oberseite und eine Hautseite aufweist, der zum Anhaften an der Haut von Menschen oder Tieren geeignet ist.

10. Medizinische Elektrode nach einem der vorstehenden Ansprüche, wobei die medizinische Elektrode ein becherförmiges leitfähiges Substrat umfasst, das eine Innenfläche und einer Außenfläche aufweist und wobei die Sensorschicht auf die Innenfläche des becherförmigen Substrats aufgetragen ist.

11. Medizinische Elektrode nach Anspruch 10, wobei das leitfähige Substrat aus einem nicht leitfähigen Substrat hergestellt ist, auf dessen Innenfläche eine leitfähige Tinte aufgetragen ist.

**Revendications**

1. Électrode médicale destinée à une surveillance de biopotentiel et/ou un enregistrement de signaux biopotentiels lorsqu'elle est fixée à la peau d'un être humain ou d'un animal, ladite électrode médicale comprenant

   - un élément de capteur comprenant une couche de capteur, et
   - une couche conductrice d'ions en contact avec le côté peau de la couche de capteur dans l'élément de capteur, ladite couche conductrice d'ions comprenant des ions chlorure,
   dans laquelle la couche de capteur comprend de l'argent (Ag) et du chlorure d'argent (AgCl) et dans laquelle la densité de surface effective d'AgCl, $\rho$A, AgCl, à la surface de la couche de capteur est déterminée par l'équation :

$$\rho_{A,AgCl} = C \cdot t_{app} \cdot \frac{1}{A_{capteur}}$$

   dans laquelle

   • pA, AgCl est la densité de surface effective d'AgCl à la surface de la couche de capteur en mg/mm$^2$,
   • C est une constante de 2,97*10$^{-10}$ g/s,
   • t est le temps de port en secondes, et
   • A$_{capteur}$ est la surface de la couche de capteur en mm$^2$, et

   dans laquelle la surface de la couche de capteur est d'au plus 100 mm$^2$ et la densité de surface effective d'AgCl est

   - d'au moins 0,00026 mg/mm$^2$ lorsque la durée de port est réglée sur 1 jour, ou
   - d'au moins 0,00051 mg/mm$^2$ lorsque la durée de port est réglée sur 2 jours, ou
   - d'au moins 0,00077 mg/mm$^2$ lorsque la durée de port est réglée sur 3 jours, ou
   - d'au moins 0,00103 mg/mm$^2$ lorsque la durée de port est réglée sur 4 jours, ou
   - d'au moins 0,00129 mg/mm$^2$ lorsque la durée de port est réglée sur 5 jours, ou
   - d'au moins 0,00154 mg/mm$^2$ lorsque la durée de port est réglée sur 6 jours, ou
   - d'au moins 0,00180 mg/mm$^2$ lorsque la durée de port est réglée sur 7 jours, ou
   - d'au moins 0,00206 mg/mm$^2$ lorsque la durée de port est réglée sur 8 jours, ou
   - d'au moins 0,00231 mg/mm$^2$ lorsque la durée de port est réglée sur 9 jours, ou
   - d'au moins 0,00257 mg/mm$^2$ lorsque la durée de port est réglée sur 10 jours ou

   dans laquelle la surface de la couche de capteur est d'au plus 2000 mm$^2$ et la densité de surface effective d'AgCl est

   - d'au moins 0,000013 mg/mm$^2$ lorsque la durée de port est réglée sur 1 jour, ou
   - d'au moins 0,000026 mg/mm$^2$ lorsque la durée de port est réglée sur 2 jours, ou
   - d'au moins 0,000039 mg/mm$^2$ lorsque la durée de port est réglée sur 3 jours, ou
   - d'au moins 0,000052 mg/mm$^2$ lorsque la durée de port est réglée sur 4 jours, ou
   - d'au moins 0,000065 mg/mm$^2$ lorsque la durée de port est réglée sur 5 jours, ou
   - d'au moins 0,000078 mg/mm$^2$ lorsque la durée de port est réglée sur 6 jours, ou
   - d'au moins 0,000091 mg/mm$^2$ lorsque la durée de port est réglée sur 7 jours, ou
   - d'au moins 0,000104 mg/mm$^2$ lorsque la durée de port est réglée sur 8 jours, ou
   - d'au moins 0,000117 mg/mm$^2$ lorsque la durée de port est réglée sur 9 jours, ou
   - d'au moins 0,000130 mg/mm$^2$ lorsque la durée de port est réglée sur 10 jours.

2. Électrode médicale selon la revendication 1, dans laquelle la surface de la couche de capteur est d'au moins 10 mm$^2$, ou d'au moins 100 mm$^2$ ou d'au moins 2 000 mm$^2$ et/ou la durée de port est située dans la plage allant de 5 minutes à 10 jours.

3. Électrode médicale selon la revendication 1 ou 2, dans laquelle l'électrode médicale est adaptée à une durée de port d'au moins 5 minutes, tel que d'au moins 1 jour ou d'au moins 4 jours ou d'au moins 7 jours ou d'au moins 10 jours.

4. Électrode médicale selon l'une quelconque des revendications précédentes, dans laquelle la couche conductrice d'ions est une pâte, un hydrogel réticulé, un hydrogel visqueux non réticulé ou un adhésif conducteur d'ions.

5. Électrode médicale selon l'une quelconque des revendications précédentes, dans laquelle l'élément de capteur est un élément de capteur imprimé comprenant une couche électroconductrice en plus de la couche de capteur, ladite couche électroconductrice est soit une couche de substrat conductrice, soit un substrat non conducteur avec une couche d'encre conductrice revêtue, telle qu'imprimée, sur le substrat non conducteur.

6. Électrode médicale selon la revendication 5, dans laquelle la couche de substrat est composée de polyéthylène téréphtalate (PET), de polycarbonate (PC), de polyuréthane thermoplastique (TPU), de polyimide (PI), de polyéthylène (PE) ou de polypropylène (PP) ou de tout mélange ou toute combinaison de ceux-ci, ou la couche de substrat est formée sous la forme d'un stratifié comprenant au moins une couche composée de polyéthylène téréphtalate (PET), de polycarbonate (PC), de polyuréthane thermoplastique (TPU), de polyimide (PI), de polyéthylène (PE) ou de polypropylène (PP) ou de tout mélange ou toute combinaison de ceux-ci.

7. Électrode médicale selon la revendication 5 ou 6, dans laquelle le substrat conducteur est formé en incorporant des particules conductrices dans une couche de substrat non conductrice, où lesdites particules conductrices sont choisies parmi le groupe consistant en du carbone, tel que des particules de graphite, des paillettes et/ou des fibres, ou le substrat conducteur peut contenir une ou plusieurs particules, paillettes et/ou fibres de métal, telles que l'étain, l'argent, l'or, le cuivre, le platine, le palladium ou des alliages ou mélanges de ceux-ci.

8. Électrode médicale selon la revendication 5 ou 6, dans laquelle l'encre conductrice comprend des particules conductrices, dans laquelle lesdites particules conductrices sont choisies parmi le groupe consistant en du carbone, tel que des particules de graphite, des paillettes et/ou des fibres, ou l'encre peut contenir une ou plusieurs particules, paillettes et/ou fibres de métal, telles que l'étain, l'argent, l'or, le cuivre, le platine, le palladium ou des alliages ou des mélanges de ceux-ci.

9. Électrode médicale selon l'une quelconque des revendications précédentes, dans laquelle ladite électrode médicale comprend en outre

   - un support de dispositif, et
   - un adhésif cutané présentant un côté supérieur et un coté peau adapté pour être fixé à la peau de l'être humain ou de l'animal.

10. Électrode médicale selon l'une quelconque des revendications précédentes, dans laquelle l'électrode médicale comprend un substrat conducteur cupuliforme présentant une surface interne et une surface externe, et où la couche de capteur est appliquée sur la surface interne du substrat cupuliforme.

11. Électrode médicale selon la revendication 10, dans laquelle le substrat conducteur est composé d'un substrat non conducteur présentant une encre conductrice appliquée sur la surface interne du substrat cupuliforme.

Fig. 1

Fig. 2a

Fig. 2b

Fig. 2c

Fig. 3a

Fig. 3b

Fig. 4a

Fig. 4b

Fig. 4c

Fig. 4d

3    6a    6    8,18    12    6b    27

## Fig. 4e

6    8,18    9    27

## Fig. 4f

12    8,18    6

27

Fig. 4g

3,9    14    15

Fig. 5a

Fig. 5b

Fig. 5c

Fig. 5d

**Fig. 6a**

**Fig. 6b**

Fig. 6c

Fig. 7

**Sensor with insufficient AgCl**

**Sensor with sufficient AgCl**

Fig. 8

Potentiostat

Counter electrode | Reference electrode | Working electrode

$I = -100 \ \mu A$

Sensor V

$Cl^-$ | Electrolyte

Fig. 9

Fig. 10

Fig. 11

Fig. 12

# EP 4 344 639 B1

**Patent documents cited in the description**

- US 2019000337 A1
- EP 2314345 B1
- US 20190320923 A1
- JP 5095922 A
- GB 2341104 B